# EUROPEAN PATENT APPLICATION

(11) **EP 4 652 933 A1**
(43) Date of publication of application: **26.11.2025**
(21) Application number: 24176952.0
(22) Date of filing: 21.05.2024
(51) Int. Cl.: A61B 5/291, A61B 5/293, A61B 5/00, A61N 1/05, A61N 1/372, A61B 5/262, A61N 1/00

(54) **ARRANGEMENT FOR INSERTING AN IMPLANT DEVICE INTO BIOLOGICAL TISSUE**

(71) Applicant: ETH Zurich, 8092 Zurich (CH)
(72) Inventor: YANIK, Mehmet, 8117 Fällanden (CH); YASAR, Tansel Baran, 8050 Zürich (CH); GOMBKÖTO, Peter, 8052 Zürich (CH); VON DER BEHRENS, Wolfger, 8712 Stäfa (CH); ÖZIL, Eminhan, 8050 Zürich (CH)

(57) **Abstract**

An arrangement (1) for inserting an implant device (2) into biological tissue (4) comprises at least one implant device (2), and at least one insertion device (3). The insertion device (3) is configured to penetrate biological tissue (4). The implant device (2) and the insertion device (3) are configured to couple to one another via an implant-insertion coupling. The implant device (2), when being coupled to the insertion device (3) via the implant-insertion coupling, is insertable into the biological tissue (4) via the insertion device (3). The implant device (2) comprises at least one bundle (5) of fibers (6, 6a, ...), and wherein the fibers (6, 6a, ...) are coupled to one another via a fiber-fiber coupling, the implant-insertion coupling being different from the fiber-fiber coupling.

## Description

### TECHNICAL FIELD

The project leading to this invention has received funding from the European Research Council (ERC) under the European Union's Horizon 2020 research and innovation program (grant agreement No 818179). The present invention relates to an arrangement for inserting an implant device into biological tissue according to claim 1 and to a method of manufacturing such an arrangement according to claim 15.

### PRIOR ART

Approximately 15% of the world's population suffers from at least one neurological disorder (World Health Organization, 2006), such as epilepsy, depression, anxiety, quadriplegia, and dementia. While some of these disorders can be treated or ameliorated through pharmaceutical or non-invasive electromagnetic treatments, diagnosing and treating other cases require more invasive bioelectronic interfaces.

In many cases, a large number of patients would benefit from the surgical implantation of intracranial electrode arrays. Common conditions, such as para- or quadriplegia, Parkinson's disease, epilepsy, and depression, already show the therapeutic potential of acute or chronic electrode implantation. For example, approximately 7.5 million patients with quadriplegia due to spinal cord injury or amyotrophic lateral sclerosis (ALS) require long-term (>10 years long) brain implants in their motor cortices that can decode their brain signals to interact with their surroundings through robotic arms or communication systems. Similarly, in patients with severe, drug-resistant Parkinson's disease (around 10 million worldwide), electrical stimulation of deep subcortical brain areas is the most effective solution to ameliorate the tremors that interfere with life quality.

In other cases, 10% of the 50 million epilepsy patients in the world need surgical removal of the brain tissue causing epileptic seizures, which is guided by the electrophysiological monitoring of the potential targets prior to the surgery. Around 100 million people (approximately 1.2% of the world population) have treatment-resistant depression (TRD) that does not respond to drugs or psychotherapy. About $43 billion of the financial burden can be attributed to TRD in the USA alone.

In these cases, particularly in more complex neuropsychiatric disorders such as TRD or TRA, interfacing with the complex brain networks comprising multiple brain structures is necessary for the effective diagnosis and treatment of these conditions. However, this interfacing has to be done: 1) in a minimally-invasive way during device implantation and biocompatible/stable over the long term to preserve the integrity of the brain tissue; 2) with the ability to reach deep brain structures, as well as superficial ones, to comprehensively target brain networks; 3) with high-quality interfacing with single neurons, to allow precise diagnostics and treatment of pathological activity in the brain tissue at the level of the fundamental building blocks of the brain networks.

Current commercial brain implants cannot meet all these criteria, being too large, stiff, or low-resolution. Electroencephalography (EEG) is a commonly used method for recording brain activity due to its non-invasiveness. However, it provides signals with very low SNR due to the significant attenuation of the electrical signals from the brain through the skull. Electrocorticography (ECoG) arrays provide higher-SNR recordings of brain activity due to their direct placement on the brain surface. However, they can only record signals from the superficial layers of the brain. They can generally provide mixed signals from only populations of neurons since they are positioned outside of the brain tissue. However, we know that neuronal population codes require single-neuron resolution since densely interconnected neurons with a wide variety of anatomical and functional properties form the fundamental building blocks of the brain networks.

Electrode arrays that can penetrate into the brain tissue can record single-neuron activity from deeper brain areas. Most state-of-the-art penetrating electrode arrays (e.g., Utah arrays, Behnke-Fried electrodes, and Michigan probes) are made of stiff materials. In the case of deep-brain stimulation or recording electrodes, the electrode arrays consist of millimeter-scale features that can cause significant acute damage to the brain tissue upon insertion. In general, stiff electrode arrays left in the brain over several weeks/months lead to scar tissue and glial encapsulation due to the mechanical mismatch between the stiff electrode array and the soft brain tissue (DOI:10.1006/exnr.1998.6983). This significantly reduces the recording quality and capability of electrical stimulation through these electrode arrays. Furthermore, the micromotion of these electrode arrays relative to the brain tissue causes relative drift in the recordings, eliminating the possibility of targeting the same neurons over time (DOI:10.1126/science.abf4588).

To prevent the problems encountered with stiff electrode arrays, various flexible and ultra-flexible electrode array technologies have been developed. It is possible to divide the current field of such flexible electrode technologies into three general categories: 1) Flexible shanks inserted by mechanical tethering of the shanks to stiff shuttles (DOls: 10.1101/578542;10.1126/sciadv. 1601966; 10.2196/16194; 10.1002/advs.201700625); 2) Flexible shanks inserted by chemical tethering of the shanks to stiff shuttles (DOls: 10.1016/j.neuron.2018.11.002;10.1109/EMBC.2012.6346070;10.1109/ISCAS.2002.10104 17; 10.1088/0960-1317/24/6/065015; 10.1038/s41551-022-00941-y); 3) alternative electrode geometries that are optimized for packing more channels into a smaller footprint or enhancing the biocompatibility, but the delivery of the electrode array is still through the chemical tethering of the electrode array to a stiff shuttle (DOIs:10.1101/460949; 10.1126/sciadv.aav2842;10.1038/s41593-023-01267-x; 10.1038/s41467-021-26168-0) or through means that cause significant damage to the brain tissue (DOls: 10.1038/nmeth.3969; 10.1038/nnano.2015.115;10.1038/nmat4427).

The first category of electrode arrays can reach deeper brain targets without depth limitations. However, the increasing number of contacts on the shanks leads to increases in the shank dimensions, compromising the biocompatibility of the arrays. The second category of electrode arrays has limitations on the target depths due to the dissolution kinetics of the chemical tethering. While the electrode arrays in the third category enable more efficient packing of the electrode arrays in a smaller footprint, they still share the same depth limitations as those in the second category.

### SUMMARY OF THE INVENTION

It is an object of the present invention to provide an arrangement for inserting an implant device into biological tissue that overcomes the drawbacks of the prior art. In particular it is an object to provide an arrangement that is minimally invasive, scalable and depth-limitation-free.

This object is achieved with an arrangement according to claim 1. That is, an arrangement for inserting an implant device into biological tissue is provided, wherein the arrangement comprises at least one implant device, and at least one insertion device. The insertion device is configured to penetrate biological tissue. The implant device and the insertion device are configured to couple to one another via an implant-insertion coupling. The implant device, when being coupled to the insertion device in the implant-insertion coupling, is insertable into the biological tissue via the insertion device. The implant device comprises at least one bundle of fibers, and wherein the fibers are coupled to one another via a fiber-fiber coupling. The implant-insertion coupling is different from the fiber-fiber coupling.

That is, the arrangement according to the invention is configured to exhibit two coupling mechanisms, namely an implant-insertion coupling that can be established between the implant device and the insertion device, and a fiber-fiber coupling that is established between fibers of the bundle of fibers.

In the fiber-fiber coupling, the fibers can be temporarily coupled to one another. Additionally or alternatively, the bundle of fibers can at least partially be coated with at least one coating, and wherein said coating provides the fiber-fiber coupling among the fibers of the bundle. The coating is preferably exclusively applied on the bundle of fibers and does not coat or otherwise encapsulate the insertion device. This separates the fiber-fiber coupling from the implant-insertion coupling mechanism. The coating preferably is biodegradable and/or configured to dissolve and/or configured to be digested upon insertion of the implant device into the biological tissue. That is, once the bundle of fibers is inserted into the biological tissue, said coating is preferably dissolved and/or digested. This leaves the fibers mechanically independent of each other, granting them maximal mechanical compliance with the biological tissue. The coating preferably comprises or consists of at least one of: polyethylene glycol, silk fibroin, polyvinylchloride, polylactic acid, polyacrylic acid or mixtures thereof.

However, it is likewise conceivable that in the fiber-fiber coupling, the fibers are permanently coupled to one another. Additionally or alternatively, the fibers can comprise one or more bridging elements tethering the fibers to one another, and wherein said bridging elements provide the fiber-fiber coupling among the fibers. To this end at least part of the fibers can comprise one or more bridging elements along their longitudinal direction. The bridging elements preferably are an integral part of the fibers, i.e. formed on the fibers and/or while manufacturing the fibers, and/or comprise or consist of at least one flexible material and/or at least one polymer, and particularly preferably comprise or consist of the same material as the fibers, see also explanations made further below with respect to conceivable the materials of the fibers and the method of manufacturing the arrangement, in particular the implant device.

In any case, the fibers in the bundle can be strongly kept together during the implantation by the fiber-fiber coupling tethering, and the implant device is additionally coupled to the insertion device by the implant-insertion coupling. This allows the bundle of fibers to be implanted as if they were a single thread or the like. This eliminates the risk of the fibers falling apart during implantation and provides reliable insertion into any depth in the biological tissue such as brain tissue.

A length of the bridging elements is preferably smaller than a length of the fibers with respect to their longitudinal direction. In particular, a ratio between the length of a bridging element with respect to the length of a fiber preferably is 1:100 or smaller. Additionally or alternatively, in the final state, the bundle of fibers preferably has an extension along a first spatial direction of 50 micrometer or smaller, and along a second spatial direction 50 micrometer or smaller for a bundle comprising about 60 fibers, such as 64 fibers.

In the event of such a permanent coupling of the fibers no coating is required to keep the bundle together since the bridging elements between the fibers serve that purpose.

Hence, the bundle of fibers preferably comprises or consists of a plurality of fibers that are permanently or temporarily coupled to each other either via the fiber-fiber coupling, in particular via the bridging element(s) or via the coating.

A conceivable number of fibers in the bundle is 20 fibers or more, for example 50 fibers or 100 fibers. Of course, there may be fewer or more fibers.

The individual fibers are preferably elongated elements that extend along a longitudinal direction. Preferably, the fibers each have an initial end and a terminal end, which are opposite each other with respect to the longitudinal direction of the fiber. The initial end can be a free end or can be connected to one or more other fibers via one or more bridging elements and/or can be connected to an insertion-support element, see further below. The terminal end can be a free end or can be connected to one or more other fibers via one or more bridging elements and/or can comprise a coupling element that serves the purpose of coupling the implant device to the insertion device in the implant-insertion-coupling, see further below.

A length of the fiber along the longitudinal direction of the fiber can be in the millimeter range such as a few millimeters or in the centimeter range, for instance 2 centimeter or more. Of course, the length can be shorter or longer and depends in particular on a penetration depth of the implant device into the biological tissue. The penetration depth in turn depends on the type or environment of the biological tissue. For example, the biological tissue may be soft and/or sensitive biological tissue such as a brain from a mammal such as a human or a rat. Since the skull and brain of the rat are smaller than the skull and brain of the human, the desired penetration depth and consequently a length of the fibers in the arrangement for inserting the implant device into biological tissue of the rat is preferably smaller than for the human.

A thickness of the fiber along a thickness direction of the fiber running perpendicularly to the longitudinal direction of the fiber is preferably in the micrometer range, for instance 20 micrometer or less, such as 10 micrometer or less.

A width of the fiber along a width direction of the fiber running perpendicularly to the longitudinal direction of the fiber and perpendicularly to the thickness direction of the fiber preferably is in the micrometer range, for instance 50 micrometer or less, such as 25 micrometer or less.

The bundle of fibers is preferably transferable from a precursor state into a final state that differs from the precursor state. In the precursor state, the bundle of fibers preferably has a two-dimensional shape, and in the final state, the bundle of fibers preferably has a three-dimensional shape. Additionally or alternatively, the implant device is preferably insertable into the biological tissue via the insertion device in the implant-insertion coupling with the bundle of fibers being in the final state.

The bundle of fibers having a two-dimensional shape preferably means that the fibers are arranged within a (fictitious) two-dimensional plane. The two-dimensional plane preferably is spanned by a first spatial direction and a second spatial direction, the first and second spatial direction running perpendicularly to one another. The first spatial direction can also be referred to as x-direction and the second spatial direction can also be referred to as y-direction.

The bundle of fibers having a three-dimensional shape preferably means that the fibers are arranged within a three-dimensional space. The three-dimensional space is preferably spanned by the first spatial direction, the second spatial direction, and a third spatial direction running perpendicularly to the first and second spatial directions. The third spatial direction can also be referred to as z-direction.

For instance, in the Euclidian space the bundle having the two-dimensional shape can be seen as having fibers that are arranged within an x-y-plane, and the bundle having the three-dimensional shape can be seen as having fibers that are arranged within the x-y-z-space, with the x-direction, the y-direction and the z-direction running perpendicularly to one another.

Hence, the transformation of the bundle of fibers from the precursor state into the final state preferably corresponds to a movement or an expansion of the fibers from a two-dimensional arrangement into a three-dimensional arrangement and/or movement or an expansion of the fibers in the third dimension.

Additionally or alternatively, the bundle of fibers having a two-dimensional shape preferably have an aspect ratio of the length of the fibers along the longitudinal direction of the fibers that is at least one order of magnitude, preferably at least two order of magnitudes such as three orders of magnitude larger than a thickness of the fibers along the thickness direction of the fibers and/or than a width of the fibers along the width direction of the fibers.

As will be outlined in greater detail below, the bundle of fibers in its precursor state is preferably manufactured by a conventional MEMS (Micro Electro Mechanical System) fabrication technique that allows mass production of implant devices on wafers, in particular by building multiple thin layers on top of each other. This allows the fabrication of the bundle of fibers being of a two-dimensional shape as well as the fabrication in an economically scalable way. The bundle of fibers in the final state preferably has a three-dimensional shape in order to improve any interactions between the implant device and the biological tissue such as a brain tissue that consists of complex circuits of neurons that are distributed in the three-dimensional space.

A variety of two-dimensional shapes and three-dimensional shapes of the fibers in the precursor state and the final state are conceivable. For instance, in the precursor state, the fibers preferably extend parallel to one another and/or along a longitudinal direction of the bundle of fibers. The longitudinal direction of the bundle of fibers preferably runs parallel to the longitudinal directions of the fibers. This means that the fiber bundle in the precursor state can have an elongated shape and/or the fibers of said bundle can have an essentially straight shape extending along the longitudinal direction of the bundle. However, other shapes of the fibers in the precursor state are just as conceivable, such as a non-straight shape, for example in a whorled pattern. For instance, the fibers of the bundle can be arranged in the form of a spiral, wherein the fibers of the bundle may be arranged in the form of a curve on the aforementioned two-dimensional plane that winds around a fixed center point at a continuously increasing or decreasing distance from that point. In other words, the fibers in the precursor state can be arranged as a flat curve that extends in its plane in both the perpendicular x- and y-directions. In particular, in the precursor state the fibers are preferably arranged in any whorled shape that allows to densely pack fibers. In particular, and as will be explained below, such a shape of the fibers allows to efficiently pack long fibers into as small of an area as possible in a two-dimensional plane or on a two-dimensional surface, respectively.

The final state preferably depends on the precursor state, whereby the transition of the fibers from the precursor state to the final state essentially involves a movement or an expansion along the third dimension, or in the case of an x-y-movement or x-y-expansion of the fibers in the precursor state, along the z-direction. For example, in a bundle with parallel fibers in the precursor state, at least some of said straight fibers can move away or be moved away from the two-dimensional plane or the x-y plane so that these fibers also extend along the z-direction. In a bundle with fibers arranged in a curve such as a spiral in the precursor state, at least part of the fibers can move away or can be moved away from the two-dimensional plane or the x-y-plane so that these fibers in the final state form a three-dimensional curve that rotates around an axis at a constant or continuously varying distance while moving parallel to the axis. An example of such a three-dimensional final state is a helix.

In summary, a two-dimensional bundle of fibers can be said to transform into a three-dimensional bundle of fibers. Regardless of an arrangement of the fibers in the precursor state, a length of the fibers of a bundle can be the same or different from one another.

The bundle of fibers is preferably transferable from the precursor state into the final state via the insertion device, preferably upon coupling of the insertion device to the bundle of fibers in the implant-insertion coupling and movement of the insertion device with respect to the implant device. The insertion device is preferably movable with respect to at least part of the implant device. For instance, and as will be explained further below, the insertion device and the implant device may each have at least one coupling element which are configured to be coupled to one another in the implant-insertion coupling, and wherein a movement of the insertion device with respect to the implant device can transmit the movement via the coupled coupling elements to the implant device, in particular to one or more of the fibers of the bundle of fibers, wherein the movement results in a movement or an extension of the fibers in the third dimension or along the z-direction, respectively. The bundle of fibers can thus be transferred from the precursor state into the final state via the insertion device, in particular when the insertion device is coupled to the bundle of fibers and is moved relative to the bundle of fibers. As will be explained in more detail below, the insertion device and the bundle of fibers each preferably have at least one coupling element that can be coupled to one another.

Additionally or alternatively, the bundle of fibers is preferably transferable from the precursor state into the final state via a movement of the fibers, in particular a deformation of the fibers such as an elastic deformation of the fibers. For this purpose, it is preferred that the fibers are movable and, in particular, deformable. As will be explained in more detail below, the fibers are preferably flexible and/or comprise one or more flexible materials.

Additionally or alternatively, the bundle of fibers is preferably transferable from the precursor state into the final state via one or more forces generated by the fibers and/or one or more forces acting on the fibers, in particular via capillary forces such as elastocapillary forces generated by the fibers and acting on the fibers. These elastocapillary forces preferably cause the aforementioned elastic deformation of the fibers. The fibers are preferably configured such that they generate these capillary forces, in particular the elastocapillary forces, upon insertion into the biological tissue. That is, after insertion of the implant device into the biological tissue, the fibers are preferably configured to coalesce into the final state by elastocapillary forces.

That is, the implant device and the insertion device are preferably separate components. The arrangement is therefore preferably a two-part or multi-part arrangement.

The bundle of fibers is preferably configured to remain in the final state. That is, the transformation of the bundle of fibers from the precursor state into the final state is preferably irreversible.

The bundle of fibers preferably comprises at least one releasing-receiving element that is configured to release into the biological tissue a signal being configured to stimulate the biological tissue and/or a diagnostic agent and/or a therapeutic agent. Additionally or alternatively, the at least one releasing-receiving element is preferably configured to receive a signal being associated with the biological tissue. To this end it is conceivable that two or more separate releasing-receiving elements are provided, one or more of them being configured to release a signal and/or a diagnostic agent and/or a therapeutic agent, and one or more of them being configured to receive a signal being associated with the biological tissue. However, the "releasing" and the "receiving" elements are not necessarily separate. For instance, the same releasing-receiving element such as an electrode contact can be used for recording electrical signals and injecting electrical currents.

Additionally or alternatively, the bundle of fibers preferably comprises at least one transmission element being configured to transmit a signal and/or a diagnostic agent and/or a therapeutic agent between i) a releasing-receiving element and ii) at least one input-output element. The input-output element preferably is configured to input a signals and/or a diagnostic agent and/or a therapeutic agent into the transmission element and to output signals from the transmission element to an external device being configured to generate or collect a signal and/or a diagnostic agent and/or a therapeutic agent.

Hence, one or more fibers can comprise one or more releasing-receiving elements and/or one or more transmission elements. In particular, it is preferred that one or more fibers of the bundle comprises a plurality of releasing-receiving elements and/or transmission elements, wherein said plurality of elements are preferably arranged along the longitudinal direction of the fibers. A surface area of the releasing-receiving element(s) preferably is in the micrometer range, for instance ranging from 25 µm² to 2500 µm². Moreover, one or more fibers can comprise at least one input-output element as well as at least one transmission element between the releasing-receiving element(s) and the input-output element(s).

The signal being released into the biological tissue and the signal being associated with the biological tissue are preferably electrical signals. Moreover, the releasing-receiving element and the input-output element preferably are a resistive or capacitive element. Moreover, the transmission element preferably is an electrically conducting element.

Alternatively, the signal being released into the biological tissue and the signal being associated with the biological tissue are preferably optical signals. Moreover, the transmission element preferably is either i) a waveguide configured to transmit optical signals or ii) an electrically conducting element that is configured to transmit electrical signals to drive a light-emitting element of the releasing-receiving element.

Alternatively, the releasing-receiving element preferably is an outlet for releasing diagnostic or therapeutic agents into the biological tissue and/or the releasing-receiving element preferably is a sensor element for sensing an amount of diagnostic or therapeutic agents in the biological tissue and/or the transmission element preferably is a microfluidic channel or an electrically conducting element.

That is, depending on the purpose of the implant device, the releasing-receiving elements can be configured for measuring an electrical activity or a fluorescence in the biological tissue, for stimulating the biological tissue with electrical currents or light, for measuring a concentration of diagnostic or therapeutic agents in the biological tissue, or for delivering diagnostic or therapeutic agents into the biological tissue, respectively. For instance, in the case that the implant device is used for electrical measurements or stimulation, it is preferred that the input-output element is a resistive or capacitive element such as a soldering pad that is configured to transmit current or voltage to an external device. The external device could comprise a circuit board that is configured to record electrical signals and electrical stimulation. The transmission element could be a metallic wire that is configured to transmit the electrical signal between the releasing-receiving element and the input-output element. In the event that the implant device is used for optical measurements or stimulations, it is preferred that the transmission element is either a waveguide that is configured to transmit light between the releasing-receiving element as well as the input-output element, or an electrically conducting element such as a metallic wire that can provide the electrical current to drive a light-emitting element such as a light-emitting diode on the releasing-receiving element. In the former case, the input-output element preferably serves as a coupler to the external device, which here preferably is a light source. In the latter case, the input-output element preferably is a resistive or capacitive element that serves as an interface to transmit current to the external device, which corresponds here preferably to a circuit boards configured to drive the light-emitting element on the releasing-receiving element. Example implementations of optical measurement and stimulation with flexible devices are presented by Ji et al., 2018 (DOI:10.1038/s41378-018-0027-0) and Reddy et al., 2020 (DOI:10.1038/s41378-020-00186-2). In the event that the implant device is used for measuring concentrations of diagnostic or therapeutic agents in the biological tissue it is preferred that the releasing-receiving element is an outlet configured to deliver the agents or a sensing element such as an electrochemical sensor for measuring the amounts of the agents in the biological tissue. In the former case, the transmission element preferably is a microfluidic channel, the external device preferably is the source of the agents to be delivered, and the input-output element preferably is a port between the microfluidic channel and the external device. In the latter case, the transmission element preferably is an electrically conducting element such as a metallic wire that is configured to transmit current and voltage between the releasing-receiving element and the external device. The input-output element preferably is a resistive or capacitive element configured to transmit voltage/current to the external device, which preferably comprises a circuit board configured to drive electrochemical reactions at the releasing-receiving elements and collecting the resulting signals. Examples of chemical delivery/measurement with flexible devices are presented by Castagnola et al., 2022 (DOI:10.3390/bios12070540) and Mariello et al., 2023 (DOI:10.1002/adhm.202302969).

The implant device, in particular the fibers, preferably comprise or consist of at least one flexible material and/or at least one polymer. For example, the fibers can comprise or consist of polyimide, parylene C, polydimethylsiloxane, parylene N, polyethylene, polymethylmethacrylate, SU-8, polyvinylchloride, polypropylene, polytetrafluoroethylene, PEDOT, or mixtures thereof.

Additionally or alternatively, the implant device, in particular the fibers, preferably comprise or consist of a flexible and/or deformable material and/or of a material having low brittleness and/or being biocompatible. For instance, the fibers preferably comprise or consist of at least one flexible polymeric material comprising or consisting of one or more polymers that can bend, stretch, and deform without breaking. Additionally or alternatively, the fibers are preferably configured flexible because of their small geometrical dimensions.

Additionally or alternatively, the implant device, in particular the fibers, can comprise or consist of at least one metallic compound, for instance at least one metallic layer. The metallic compound, in particular the metallic layer, can comprise or consist of at least one of: gold, platinum, tantalum, palladium, iridium, titanium, aluminium, nickel, copper, magnesium, or chromium. Such one or more metallic compounds are preferred in the event of releasing-receiving elements or transmission elements being configured to transmitting/receiving electrical signals due to their low sheet resistance and compatibility with existing high-resolution MEMS fabrication techniques. When transmitting/receiving optical/chemical signals, polymers are preferred. One or more metallic compounds can also be used for manufacturing purposes, for instance as sacrificial release layers in an MEMS manufacturing process of the implant device. Metallic compounds such as gold, platinum, tantalum, palladium, titanium, iridium are preferred due to their inertness and high conductivity. These metals are preferred transmission elements in long-term implant devices. Metallic compounds such as aluminium, nickel, copper, magnesium, chromium are helpful for supporting roles in expanding the implant device from its 2D-shape into the 3D-shape due to their availability for controlled red-ox reactions. Hence, metallic compounds being used as transmission elements preferably have high conductivity, deformability and chemical inertness. Metallic compounds being used for other purposes are preferably deformable and/or available for redox reactions.

Hence, the fibers preferably comprise or consist of one or more layers of flexible material and/or of one or more polymers and/or of one or more metallic layers that are arranged above one another with respect to the thickness direction of the fibers. In the event of the implant device comprising the releasing-receiving elements, the transmission elements and the input-output elements for electrically recording and/or stimulation as mentioned earlier, it is preferred that these elements are provided by at least one of these metallic compounds, in particular metallic layers. Moreover, the at least one metallic compound, in particular metallic layer, is preferably encapsulated in one or more polymer layers.

Transmission elements in the form of waveguides or microfluidic channels are preferably formed by structuring multiple polymeric layers as it is well-known in the art.

In addition, the releasing-receiving elements are preferably coated with at least one impedance-reducing coating that is configured to reduce an electrical impedance between releasing-receiving elements and the surrounding biological tissue. The impedance-reducing coating preferably is a layer and/or comprises or consists of a polymer mixture of two ionomers such as poly(3,4-ethylenedioxythiophene) polystyrene sulfonate (PEDOT:PSS). However, other impedance-reducing coatings are likewise conceivable and are well-known in the art.

Further explanations regarding the structure and in particular the manufacturing process of the implant device and the insertion device will be provided further below.

The implant device, in particular the fibers, preferably comprise at least one contrast element being configured to generate or enhance a contrast in the biological tissue during imaging of the biological tissue with electromagnetic waves.

The contrast element preferably comprises or consists of at least one metallic compound and/or at least non-metallic compound.

Examples of conceivable metallic compounds are metallic nanoparticles such as iron oxide nanoparticles, preferably provided as one or more layers, or one or more metallic layers. For instance, a nickel or gadolinium layer could be deposited in the vicinity of a releasing-receiving element in the form of an electrode. It is likewise conceivable that a layer of metallic nanoparticles is provided by a layer of nanoparticles patterned on a surface, which can be nonuniform based on the interaction forces between the nanoparticles. Examples of conceivable non-metallic compounds are metal-free MRI contrast agents such as nitroxide-based compounds, or compounds that are based on isotopes of carbon or fluor (see publication DOI: 10.1021/acscentsci.7b00253 and the literature cited there).

The contrast element is preferably provided at least partially on the transmission element and/or the releasing-receiving element(s) in order to generate contrast in the biological tissue during imaging with electromagnetic waves. The contrast element is preferably provided, for instance deposited, in the form of a unique identifier pattern on each implant device such, that the implant device can be identified in the biological tissue during imaging with electromagnetic waves. That is, the unique identifier pattern of contrast enables a distinction of each implant device when multiple implant devices are in the biological tissue.

The insertion device preferably is stiff and/or comprising a Young's modulus in the range of 10 MPa to 500 GPa.

Additionally or alternatively, the insertion device preferably is retractable from the implant device after insertion of the implant device into the biological tissue or dissolvable upon the insertion of the implant device into the biological tissue in the implant-insertion coupling.

Additionally or alternatively, the insertion device preferably is water-soluble or water-insoluble. That is, the insertion device can be retractable from the implant device after insertion of the implant device into the biological tissue. To this end it is preferred that the implant device and the insertion device are removably coupled to one another in the implant-insertion coupling via respective coupling elements, see further below. In this case it is further preferred that the insertion device is water-insoluble. However, it is likewise conceivable that the insertion device is not removed from the implant device after insertion of the implant device into the biological tissue but remains inserted together with the implant device. To this end it is preferred that the insertion device is dissolvable and/or water-soluble and/or biodegradable.

Additionally or alternatively, the insertion device can comprise or consist of at least one metal compound and/or at least one non-metal compound and/or at least one metalloid compound and/or at least one ceramics compound and/or at least one biodegradable compound and/or at least one polymer. Examples of conceivable metal compounds are tungsten, iron, magnesium, titanium and alloys thereof and/or oxides thereof. An example of a conceivable alloy is an alloy of iron, for instance stainless steel. Examples of conceivable non-metal compounds are carbon-compounds such carbon fibers. Examples of conceivable metalloid compounds are silicon compounds, for instance silicon oxides or silicon carbides. Examples of conceivable ceramics compounds are inorganic, metallic oxide, nitride, or carbide compounds, for instance a silicate ceramic such as silicon carbide ceramic. Examples of conceivable biodegradable compounds are soluble carbohydrate compounds, for instance a sugar compound such as a disaccharide, for instance maltose. Examples of conceivable polymers are biocompatible and/or synthetic and/or hydrophilic polymers, for instance a polyether compound such as polyethylene glycol.

The insertion device preferably is an elongated device extending along a longitudinal direction. The insertion device preferably has an initial end and a terminal end, which are opposite each other with respect to the longitudinal direction of the insertion device. A length of the insertion device along the longitudinal direction of the insertion device preferably is in the millimeter range such as 1-2 millimeter or in the centimeter range, for instance 1 centimeter or more. Of course, the length can be shorter or longer and again depends in particular on the penetration depth of the implant device into the biological tissue. A thickness of the insertion device along a thickness direction of the insertion device running perpendicularly to the longitudinal direction of the insertion device is preferably in the micrometer range, for instance 50 micrometer or more, such as 100 micrometer or 150 micrometer. A width of the insertion device along a width direction of the insertion device running perpendicularly to the longitudinal direction of the insertion device and perpendicularly to the thickness direction of the insertion device preferably is in the micrometer range, for instance 50 micrometer or more, such as 100 micrometer or 150 micrometer.

The initial end is preferably connectable or connected to an insertion-support element and/or an alignment element and/or a handling element. The arrangement thus preferably additionally comprises at least one insertion-support element and/or at least one alignment element and/or at least one handling element.

The insertion-support element preferably serves the purpose of supporting the insertion device, and in particular a plurality of insertion devices, see further below. For example, one or more insertion devices can be arranged on the insertion-support element, for example attached to an outer surface of the insertion-support element via their initial ends.

Preferably, the insertion device extends angularly, in particular at a right angle, away from the insertion-support element, in particular from an outer surface thereof, when seen from the initial end in the direction of the terminal end of the insertion device.

The alignment element preferably serves the purpose of controlling extension angle(s) of the insertion device(s), see also further below. For example, the alignment element may be a plate or the like to which the insertion device is preferably connected to via its initial end or connectable to via the insertion-support element. Preferably, the insertion device is arranged at an angle, preferably at a right angle, with respect to the alignment element and in particular with respect to an outer surface thereof, when seen from the initial end in the direction of the terminal end of the insertion device.

The insertion device may be connectable to or connected to a handling element. The handling element preferably serves the purpose of facilitating and handling of the insertion device and thus of the implant device. For example, the handling element may be a handle or the like that can be grasped by a user such as a surgeon and that allows manual insertion of the insertion device into the biological tissue. Equally conceivable, however, is a handling element that allows the insertion device to be connected to a robotic arm or the like, so that the insertion device can be inserted mechanically or computer-controlled.

It should be understood that the insertion device can be connected to the alignment element and the handling element. In this case, it is preferred that the insertion device is connected to the alignment element, in particular via its initial end and/or via the insertion-support element, and that the alignment element is in turn connected to the handling element. The alignment plate can be removable from the insertion device or it can remain on the insertion device. In the latter case, the alignment plate can be seen as being part of the insertion device and/or is preferred in the event of the bundle of fibers being arranged in their precursor states in an array as mentioned initially.

The terminal end of the insertion device can be a free end that preferably further comprises at least one a coupling element that serves the purpose of coupling the insertion device to the implant device in the implant-insertion coupling. That is, the implant device preferably comprises at least one coupling element and the insertion device comprises at least one coupling element that are configured to couple to one another in the implant-insertion coupling. The coupling element of the implant device and the coupling element of the insertion device are preferably configured to mechanically couple to one another such as to engage one another. That is, the implant device and the insertion device are preferably configured to removably couple to one another via their coupling elements in the implant-insertion coupling.

Additionally or alternatively, the coupling element of the implant device is preferably arranged and/or integrally formed on the bundle of fibers, in particular at a terminal end of a fiber. Additionally or alternatively, the coupling element of the insertion device is preferably arranged and/or integrally formed on the insertion device, in particular at a terminal end of the insertion device. In fact, at least one of the fibers preferably terminates in the coupling element, wherein the terminal end of said fiber comprises the coupling element. For example, at least one of the fibers can terminate in a coupling element in the form of a loop that is configured to accommodate and thereby engage with a coupling element in the form of a tip or needle being arranged or formed on the insertion device. The tip or needle is preferably formed by a steep taper of the insertion device when seen inwards in the direction of a central longitudinal axis of the insertion device, which ends in the terminal end of the insertion device. An inner diameter of the loop preferably is in the micrometer range, for instance 50 micrometer or less such as 25 micrometer. An outer diameter of the loop preferably is in the micrometer range, for instance 50 micrometer or more, such as 75 micrometer. A diameter of the tip or needle preferably is in the micrometer range. For instance, the diameter of the tip or needle at the terminal end of the insertion device can be 50 micrometer or less, such as 25 micrometer or less. However, smaller or larger diameters are likewise conceivable. For instance, the loop could have an outer diameter being smaller than 50 micrometer such as 10 micrometer, etc. Preferably, the diameter of the tip or needle is smaller than the inner diameter of the loop, so that the tip or needle can be inserted into the loop and coupled with it. For example, the diameter of the tip or needle can be a few micrometer smaller, e.g., the diameter of the tip or needle could be 20 micrometer, and the inner diameter of the loop could be 25 micrometer.

The coupling element of the implant device is preferably provided at a center of the implant device. In particular, in the case of an implant device which, in the precursor state, has fibers extending parallel to one another and/or along the longitudinal direction of the bundle of fibers, the coupling element is preferably arranged on one or more of central fibers of the bundle. Or, in other words, the coupling element is preferably not arranged on the outer fibers of the bundle. It is further preferred that the one or more central fibers are longer than other fibers of the bundle. When coupling the insertion device to the implant device, the insertion device is preferably moved along a longitudinal direction of the implant device until the coupling elements couple with each other. In particular, the insertion device is preferably moved along an outside of the bundle of fibers, i.e., outside or spaced from the outer fibers of the bundle, and finally engages with the coupling element(s) of the implant device. In other words, the insertion device is preferably not moved through the bundle of fibers. In this coupled state, the insertion device and the implant device are preferably non-displaceable relative to each other and are jointly insertable into the biological tissue. In the case of an implant device which, in the precursor state, comprises fibers arranged in a curve extending from or winding around a center point, it is preferred that the coupling element is preferably arranged in the region of the center point. In particular, it is preferred that the center point is formed by one or more terminal ends of the fibers, and wherein the coupling element is arranged or formed on these terminal ends. In other words, the fibers being arranged in a curve such as wound into a spiral shape are preferably wound radially outwards from their terminal ends, i.e. their initial ends preferably being spaced radially outwards from the terminal ends when viewed along a radial direction. The radial direction preferably lies in the (fictitious) two-dimensional plane within which the fibers are curved. When coupling the insertion device to the implant device, the insertion device is preferably moved along a longitudinal direction of the implant device until the coupling elements couple with each other. In particular, the insertion device is preferably guided centrally through the center point of the curved fibers and initially engages with the coupling element(s) of the implant device. If the insertion device is now moved further with respect to the implant device, it moves the fibers along with it due to the coupling of the coupling elements and thereby increasingly expands the fibers from their 2-dimensional precursor shape into the final 3-dimensional shape. In the final state, the insertion device is preferably located within the fibers of the bundle, with the fibers winding around the insertion device along the longitudinal direction of the insertion device. In this coupled state, the insertion device and the implant device are preferably non-displaceable relative to each other and are jointly insertable into the biological tissue.

It should be noted that the implant device and/or the insertion device may comprise two or more coupling elements. The two or more coupling elements of the implant device may be arranged on the same one or more fibers of the bundle and/or on different fibers of the bundle. For example, one or more fibers may have coupling elements at different positions along the fiber(s). Two or more coupling elements of the insertion device are preferably arranged at different positions along the insertion device as well. However, and as will be explained further below, it is likewise conceivable that the arrangement comprises two or more insertion devices, and wherein each insertion device comprises at least one coupling element that is configured to couple to the coupling elements of the implant device.

A number of coupling elements of the implant device can be equal to or different from a number of coupling elements of the insertion device(s).

The implant device preferably comprises a plurality of bundles of fibers. At least in the precursor states, the plurality of bundles of fibers are preferably arranged on a fiber-support element, in particular on a die of a wafer, and/or in a common plane and/or in a one-dimensional array or in two-dimensional array.

The arrangement preferably comprises a plurality of insertion devices. A plurality of bundles of fibers is preferably transferrable from their precursor states into their final states via the plurality of insertion devices and preferably simultaneously. Additionally or alternatively, the plurality of insertion devices is preferably arranged on an insertion-support element and/or in a common plane and/or in a one-dimensional array or in a two-dimensional array.

Explanations made with respect to one bundle of fibers preferably likewise apply to the plurality of fibers and vice versa. Likewise, explanations made with respect to one insertion device preferably likewise apply to the plurality of insertion devices and vice versa.

For instance, the plurality of bundles of fibers at least in their precursor state can be provided such as patterned on a fiber-support element, in particular on a die of a wafer. For the sake of completeness, it is noted that one bundle instead of a plurality of bundles of fibers could be arranged on the fiber-support element as well. The said one bundle or the plurality of bundles of fibers are preferably an integral part of the fiber-support element.

The fiber-support element preferably comprises or consists of one at least one semiconducting compound and/or at least one dielectric compound and/or at least one metallic compound and/or at least one biodegradable compound. Additionally or alternatively, the fiber-support element can be a layered structure comprising or consisting of one or more of semiconductor layers and/or one or more metallic layers and/or one or more dielectric layers and/or one or more biodegradable layers. If two or more layers are present they are preferably arranged above one another with respect to a thickness direction of the fiber-support element. Conceivable semiconducting compounds are silicon, alloys of elements in Groups III and V of periodic table such as gallium arsenide, gallium nitride, indium nitride, alloys of elements in Groups II and V of periodic table such as zinc telluride, zinc sulfide. Conceivable dielectric compounds are silicon oxide, silicon nitride, aluminium oxide. Conceivable metallic compounds are gold, platinum, copper, titanium, tantalum, iridium. Conceivable biodegradable layers are one or more of the materials the insertion device(s) can comprise or consist of that were listed earlier.

In the event of the implant device being manufactured according to a MEMS technique it is preferred that the fiber-support element comprises or consists of at least one planar material such as a die of a wafer, for instance a polyimide or silicon wafer.

The fiber-support element preferably has a planar shape and defines the common plane the plurality of bundles is arranged in.

In particular, the plurality of bundles of fibers, at least in their precursor states, are preferably arranged in a one-dimensional or two-dimensional array, and wherein the fibers of each bundle are particularly preferably arranged in a curve such as a spiral. That is, the plurality of bundles of fibers can be provided in a one- or two-dimensional array of curved fibers.

The plurality of bundles of fibers is preferably transferrable from their precursor states into their final states via the plurality of insertion devices, and in particular upon coupling of the coupling elements of the fibers with the coupling elements of the insertion devices and movement of the insertion devices with respect to the implant device, wherein the movements of the insertion devices are transmitted via the coupled coupling elements to the fibers, resulting in an extension of the fibers in the third dimension or along the z-direction, respectively.

The transformation of the plurality of bundles from the precursor states into the final states preferably occurs simultaneously.

To this end it is preferred that the plurality of insertion devices is arranged in a common plane and/or in a one-dimensional array or in a two-dimensional array and particularly preferably in accordance with the plurality of bundles of fibers. That is, if the bundles of fibers are arranged in a one-dimensional (two-dimensional) array, the plurality of insertion devices are preferably arranged in a one-dimensional (two-dimensional) array as well. It is further preferred that, when seen along a coupling direction along which the implant device, in particular the plurality of bundles, and the insertion devices are coupled to each other, the array of the insertion devices and the array of the bundles of fibers are arranged at least partially one above the other or at least partially congruent, respectively.

As mentioned earlier, the plurality of insertion devices can be arranged on an alignment element or on the insertion-support element. Said alignment element or insertion-support element preferably defines the common plane and/or the array the plurality of insertion devices is arranged in.

The arrangement can comprise at least one alignment device that comprises at least two alignment elements being arranged above one another with respect to an alignment direction. The insertion device is preferably connectable to one of the alignment elements and the implant device comprising the bundle of fibers in the precursor state is connectable to the other alignment element. The alignment elements are preferably movable with respect to one another and along the alignment direction such, that the insertion device transfers the bundle of fibers from the precursor state into the final state. To this end it is particularly preferred that a plurality of insertion devices and an implant device comprising a plurality of bundles of fibers are connectable to the alignment elements. The alignment element to which the insertion device(s) are connectable preferably corresponds to the alignment element mentioned earlier. That is, the alignment element preferably is a plate or the like comprising a surface, to which the insertion device(s) such as their initial ends or via the insertion-support element can be connected. For instance, insertion devices in the form of wires, e.g. metallic wires such as tungsten wires, can pass through guiding holes in the alignment elements and can be attached such as glued to the alignment element, preferably to the alignment element that is movable up and down or along the alignment direction, see below.

The alignment element to which the implant device is connectable preferably corresponds to a plate or the like as well, and wherein the implant device, preferably the fiber-support element comprising the bundle(s) of fibers, are preferably connectable to a surface thereof such as glued on the alignment element.

When seen along the alignment direction along which the alignment elements are movable towards and/or away from one another, the alignment elements are preferably arranged at least partially congruent. When the implant device and the insertion device(s) are connected to the alignment elements, the alignment direction preferably runs parallel to the coupling direction along which the implant device, in particular the plurality of bundles, and the insertion devices are coupled to each other.

Thus, the alignment device enables the simultaneous transformation of the plurality of bundles from the precursor states into the final states mentioned earlier in a simple manner. Furthermore, the alignment device enables a parallel adjustment of the positions of the plurality of insertion devices with respect to the plurality of bundles of fibers, i.e. enables moving all the insertion devices in parallel, along a direction perpendicular to the surface of the alignment element, so that they can expand all the bundles of fibers in their precursor state such as spirals in a 2D grid into 3D bundles simultaneously.

The alignment element being connectable to the implant device preferably comprises at least one guiding hole that is arranged to match a position of the insertion device when being connected to its alignment element such, that the insertion device is at least partially protruding though the guiding hole upon moving the alignment elements with respect to one another, whereby the insertion device is configured to transfer the bundle of fibers from the precursor state into the final state in a guided manner.

The alignment element being connectable to the implant device preferably comprises a number of guiding holes that matches a number of bundles and/or a number of coupling elements that are to be coupled to the insertion device(s). In the case of the plurality of bundles it is therefore preferred that the alignment element being connectable to the implant device comprises a plurality of guiding holes such that the insertion devices, upon movement of one or both of the alignment elements towards one another, are at least partially inserted into the guiding holes and protruding through the guiding holes with their coupling elements such, that a coupling with the coupling elements of the bundles of fibers and ultimately a movement or expansion of the bundles from the precursor states into the final states occurs in a guided manner. The at least one guiding hole preferably is a through hole that extends completely through the alignment element such as the plate.

The alignment device preferably further comprises at least one driving element being configured to drive the movement of the alignment elements, the driving element preferably being a mechanical driving element such as a screw or an electrical driving element such as a piezoelectric element or a motor. The alignment device preferably further comprises at least one frame element, wherein one or both of the alignment elements are preferably movably mounted on the frame element. Moreover, the at least one driving element is preferably also mounted to the frame element. The alignment elements and the drive element are preferably arranged and designed such that actuation of the driving element moves the alignment element(s) towards and away from each other, wherein the alignment element(s) are moved along the frame element and along the alignment direction. Thus, the alignment device further enables a guided transformation of the plurality of bundles from the precursor states into the final states mentioned.

In another aspect, a method of manufacturing an arrangement for inserting an implant device into biological tissue is provided. Said arrangement preferably corresponds to the arrangement described above. The method comprises the steps of i) providing at least one implant device, and ii) providing at least one insertion device. The insertion device is configured to penetrate biological tissue. The implant device and the insertion device are configured to couple to one another via an implant-insertion coupling. The implant device, when being coupled to the insertion device in the implant-insertion coupling, is insertable into the biological tissue via the insertion device. The implant device comprises at least one bundle of fibers, and wherein at least some of the fibers are coupled to one another via a fiber-fiber coupling.

Any statements made with respect to the arrangement per such as its implant and insertion devices per se preferably likewise apply to the method of manufacturing the arrangement and vice versa.

The implant device is preferably provided by an MEMS (Micro Electro Mechanical System) fabrication technique that is well-known in the art and/or by:
(1) patterning at least one metallic compound such as titanium or gold on at least one substrate layer, in particular on a die of a wafer preferably comprising or consisting of a polymer, e.g. a polyimide substrate layer or die, to produce one or more transmitting elements for instance in the form of wires, one or more releasing-receiving elements for instance in the form of electrode pads, and one or more input-output elements for instance in the form of soldering pads;
(2) coating the transmitting, releasing-receiving and input-output elements with at least one insulating layer, preferably a polymer layer; and
(3) etching the insulating layer being arranged above the input-output elements with respect to the thickness direction and etching the substrate layer and the insulating layer so as to form at least one bundle comprising a plurality of fibers that comprises at least one coupling element for instance in the form of a loop that is configured to couple with a coupling element of the insertion device in the implant-insertion coupling and that optionally further comprises at least one bridging element between the fibers providing the fiber-fiber coupling.

Steps (1) to (3) are preferably carried out in this order and preferably one after the other.

That is, in step (3) the bridging elements of the fiber-fiber coupling and the coupling elements such as the loops of the implant-insertion coupling can be formed. To this end it is preferred that two etching processes are done in once though: 1) etching of the insulating layer to enable electrical access to the input-output elements; 2) etching the (polyimide) substrate layer and insulating layer in order to form the fibers, the bridging elements and the loops.

It should be noted that the temporary fiber-fiber coupling by means of the coating that coats the bundle of fibers could be provided instead of the fiber-fiber coupling by means of the bridging elements. In this case it is preferred to apply said coating to the bundle for instance by dip-coating or painting. However, it is likewise conceivable that said coating is applied in the above-described MEMS fabrication as well.

A shape of the bundle of fibers can be determined by the shape the constituents of the implant device are provided. For instance, bundles of fibers comprising parallel fibers in the precursor state can be manufactured by patterning the metallic compound in step (1) along parallel lines on the substrate layer. Bundles of fibers comprising fibers being arranged in a curve such as a spiral can be manufactured by patterning the metallic compound in step (1) in a curve such as a spiral on the substrate layer.

The method can further comprise the steps of coating the releasing-receiving elements with at least one impedance-reducing coating element, in particular with at least one coating layer. Said steps preferably comprise:
(4) Applying at least one sacrificial layer after the etching of step (3);
(5) Etching the sacrificial layer and the insulating layer on the releasing-receiving elements;
(6) Applying at least one impedance-reducing coating layer preferably by spin-coating; and
(7) Removing the sacrificial layer, wherein the releasing-receiving elements are coated with the impedance-reducing coating layer.

The impedance-reducing coating layer preferably is PEDOT:PSS, although other impedance-reducing coating elements can likewise be used. It is furthermore preferred that the impedance-reducing coating layer is baked after the spinning.

Additionally or alternatively, the method can further comprise the step of fixing the fibers, in particular the fibers arranged in an array such as in spiral arrays, during their manufacturing. To this end it is preferred that a fixation layer is deposited between the fiber-support element and the substrate layer. The fixation layer can be an aluminium layer helps fix the fibers in the precursor state such as spiral-shaped fibers stably on the substrate layer during the fabrication. Said fixation or sacrificial layer is preferably removed shortly before the process of expanding the two-dimension precursors into the 3D bundles, so that they are mechanically free to expand.

It should be noted that the above steps can be performed for one or multiple layers. For instance, multiple metallic layers can be provided to be able to fit transmitting elements and fibers more compactly. In that case it is preferred to etch the polymer between the two layers at desired locations for via connections and the like.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

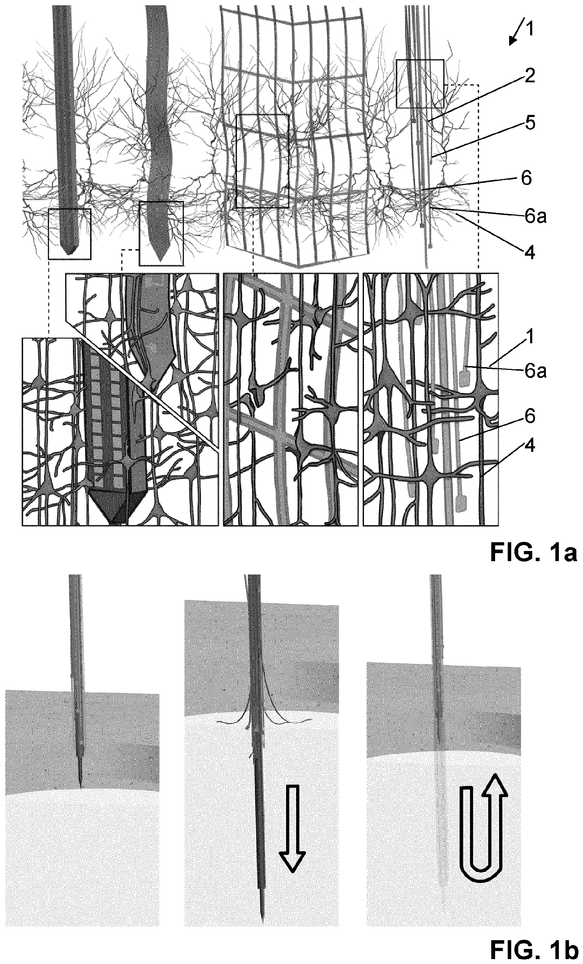

- Fig. 1: shows a comparison of an arrangement comprising an implant device and an insertion device of the invention with the state of the art. A: Interaction of the stiff electrode array (first one from the left), flexible shank (second one from the left), mesh electrode array (third one from the left) of the state of the art and an arrangement of the invention (fourth one from the left). The zoomed-in views of the boxes in the top row are provided in the bottom row. B: In the case when fibers are encapsulated in a chemical coating together with an insertion device (left), either the coating dissolves too rapidly and the fibers get separated prematurely from the insertion device (middle) or the coating is too strong and the fibers get stuck to the insertion device.

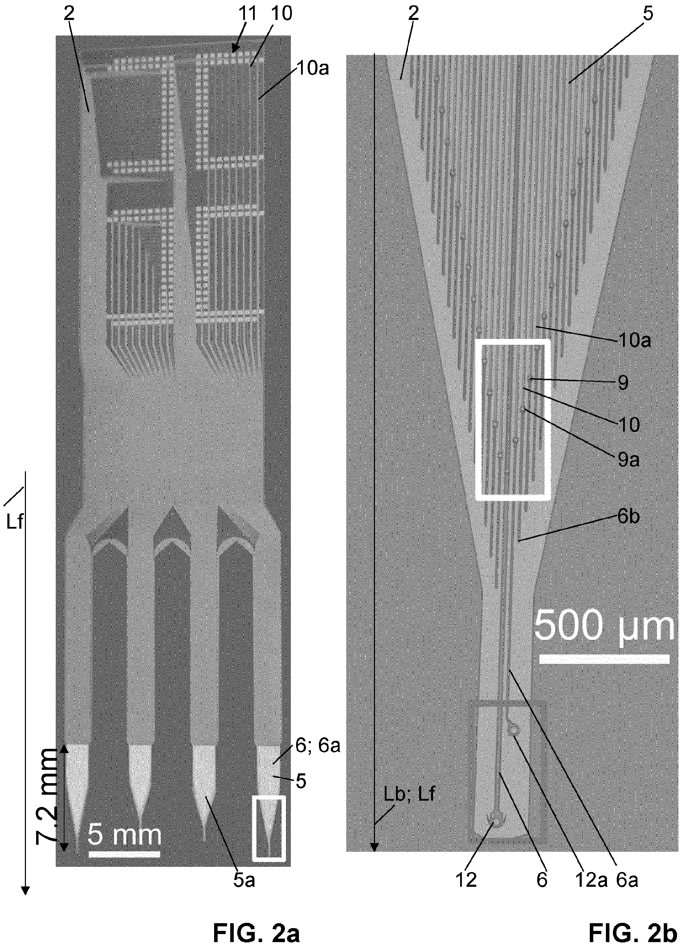

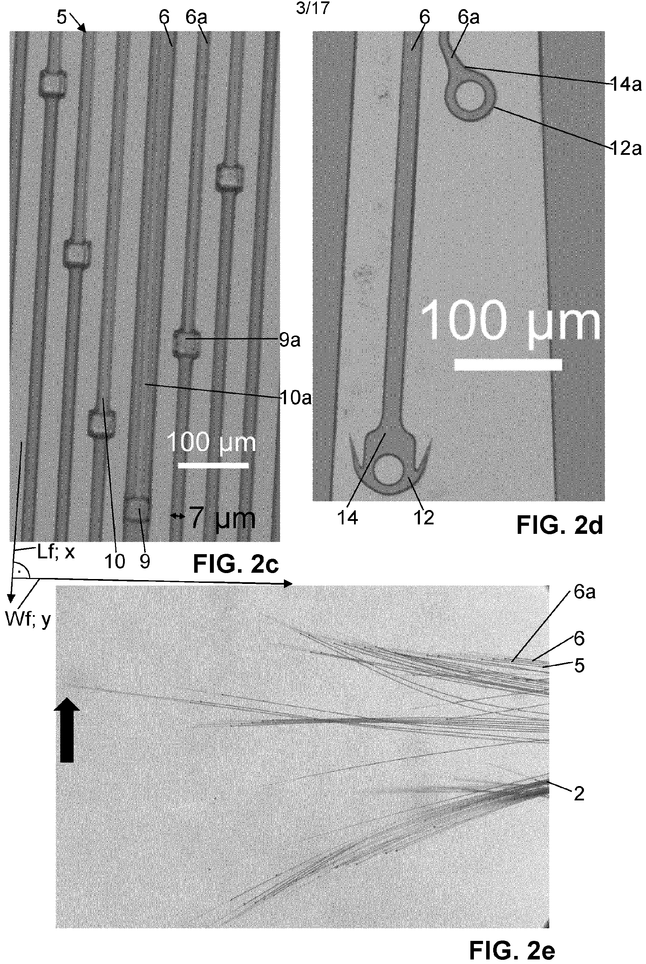

- Fig. 2: shows an implant device comprising of multiple bundles of linear fibers. A: The overview of the implant device on a wafer. B: The zoomed-in view of the white box in Fig. 2A shows a close-up view of one of the bundles. C: The zoomed-in view of the white box in Fig. 2B shows the fibers and releasing-receiving elements in the form of contacts on them. D: The zoomed-in view of the black box in Fig. 2C shows a coupling element in the form of a loop at the end of the fiber. E: The fibers freely float in water after removal from the wafer. The arrow indicates the position of the loop;

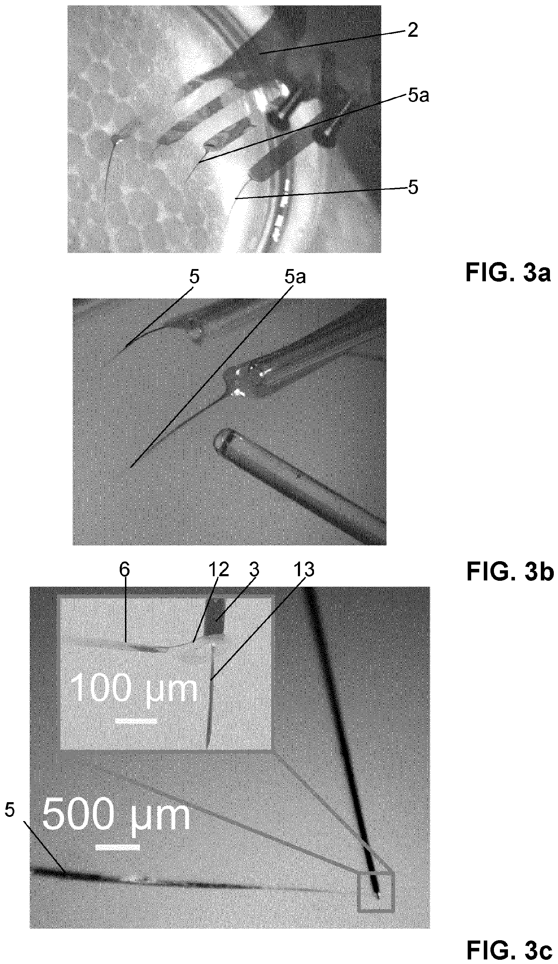

- Fig. 3: shows steps of arranging the implant device and an insertion device, wherein the implant device comprises a bundle of linear fibers. A: The bundles of fibers are coated with 2% polyethylene glycol solution in water. B: The bundles of fibers are coated with silk fibroin solution. C: The coupling element at the tip of an insertion device is coupled with the coupling element at the tip of a bundle of fibers. The inset shows the zoomed-in view of the coupling between the two coupling elements.

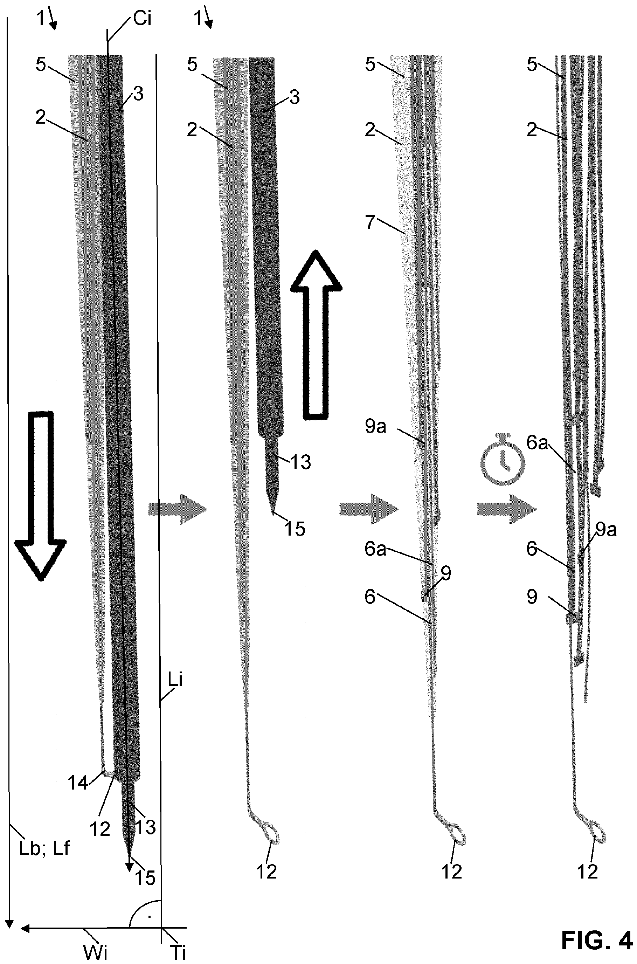

- Fig. 4: shows steps of an insertion method of an arrangement comprising the implant device and an insertion device, wherein the implant device comprises a bundle of linear fibers;

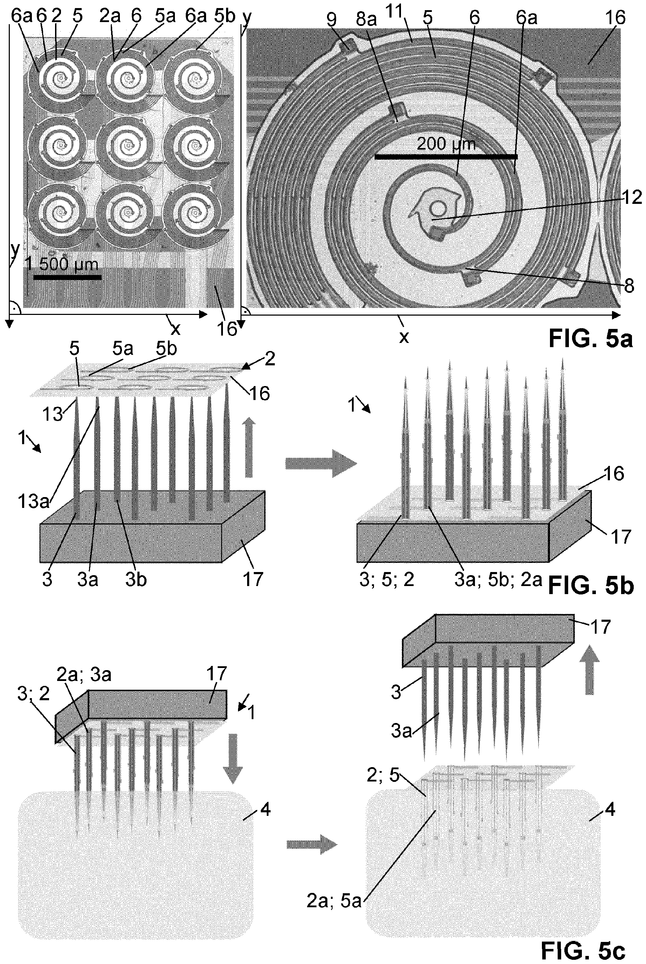

- Fig. 5: A shows: A 3x3 array of bundles of fibers in a precursor state, wherein the fibers are shaped as spirals. B: Parallel expansion of the two-dimensional spirals in the precursor state into a final state of the bundles of fibers, wherein the bundles of fibers are arranged in an array of three-dimensional bundles with the help of an array of stiff insertion device. C: The parallel insertion of the array of three-dimensional bundles into soft tissue biological tissue;

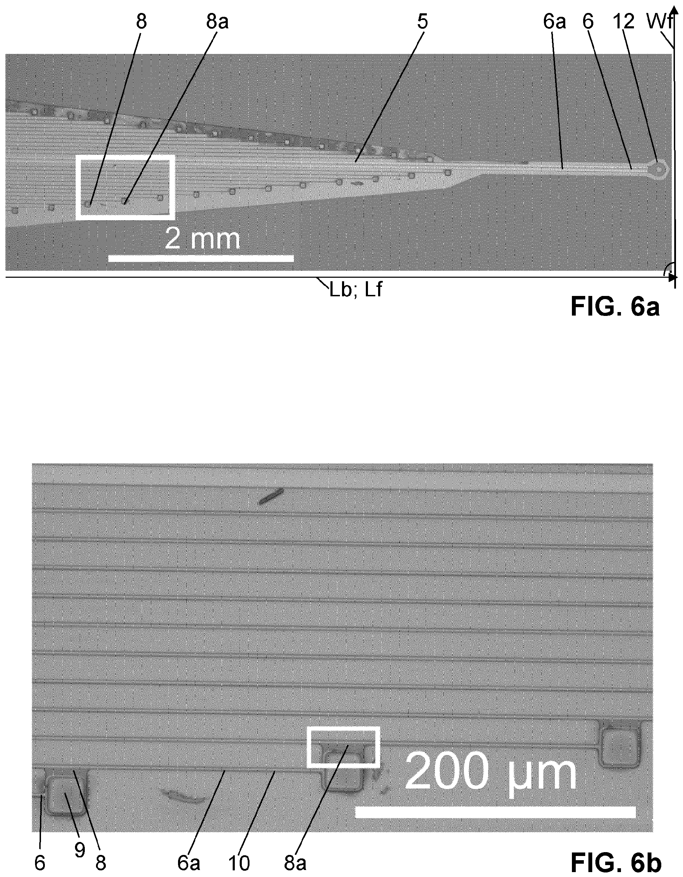

- Fig. 6: A shows: An alternative embodiment of the implant device. B: A zoomed-in view of the white box in Fig. 6A, highlighting bridging elements between two neighboring fibers in a white box;

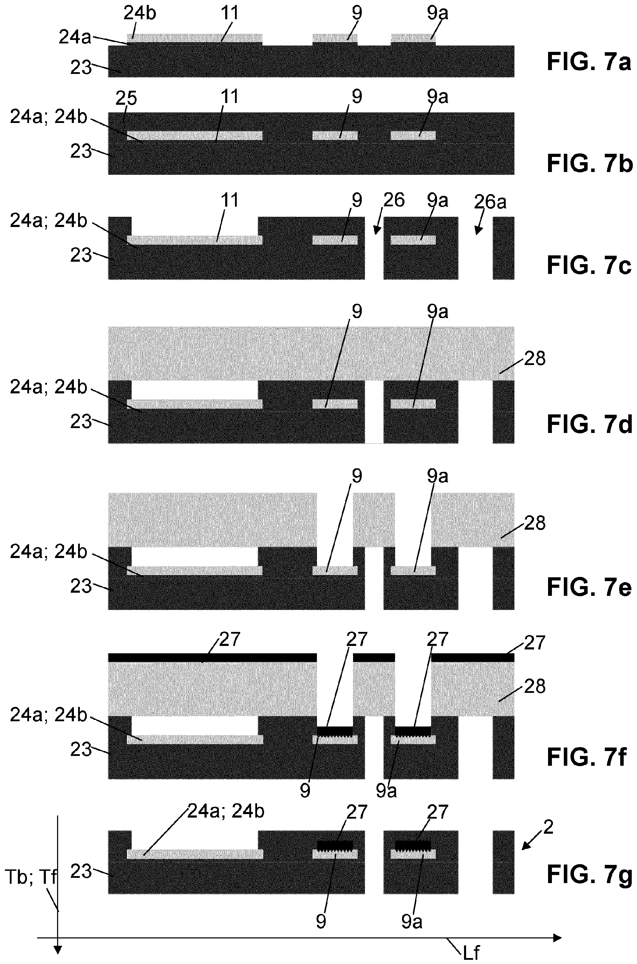

- Fig. 7: shows manufacturing steps of an implant device comprising a bundle of fibers being arranged parallel in the precursor state. A: Patterning metallic compounds on a substrate layer of a wafer to define transmission elements in the form of wires and releasing-receiving elements in the form of electrode pads. B: Coating of an insulation layer on the metallic compounds. C: Etching of the insulating layer above the releasing-receiving elements and between so as to form fibers. D: Coating a sacrificial layer on the wafer. E: Etching of the sacrificial layer and insulating layer on the releasing-receiving elements. F: Spin-coating the wafer with a mixture of PEDOT:PSS, ethylene glycol, 3-glycidyloxypropyltrimethoxysilane, and 4-dodecylbenzene sulfonic acid. G: Removal of the sacrificial layer from the wafer;

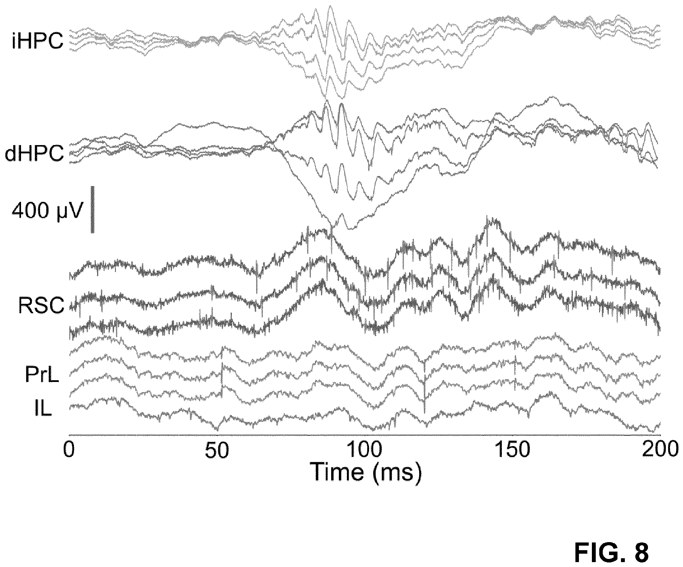

- Fig. 8: shows simultaneous high-quality population and single-neuron recordings from multiple brain areas with the implant device;

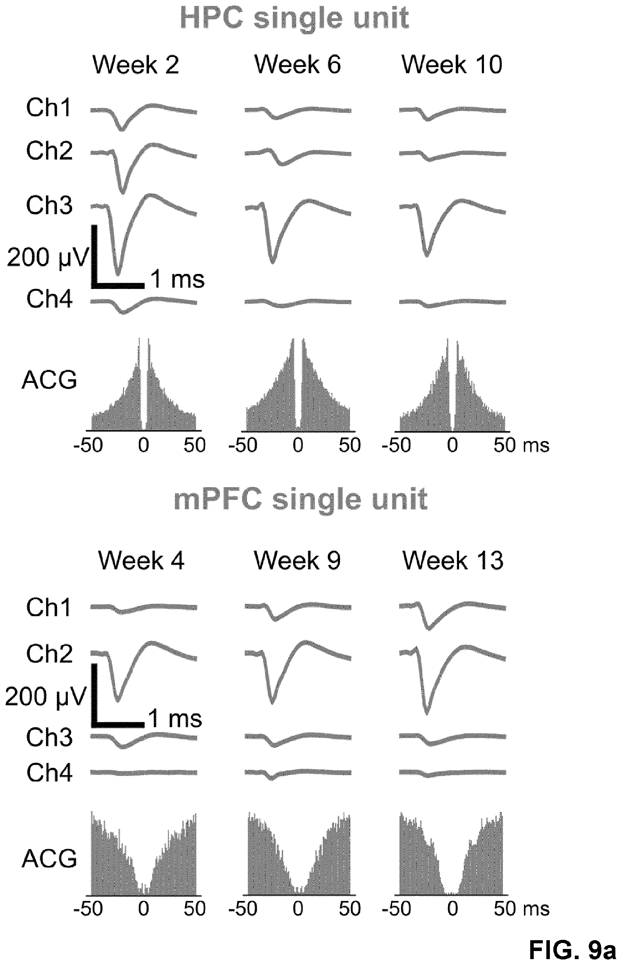

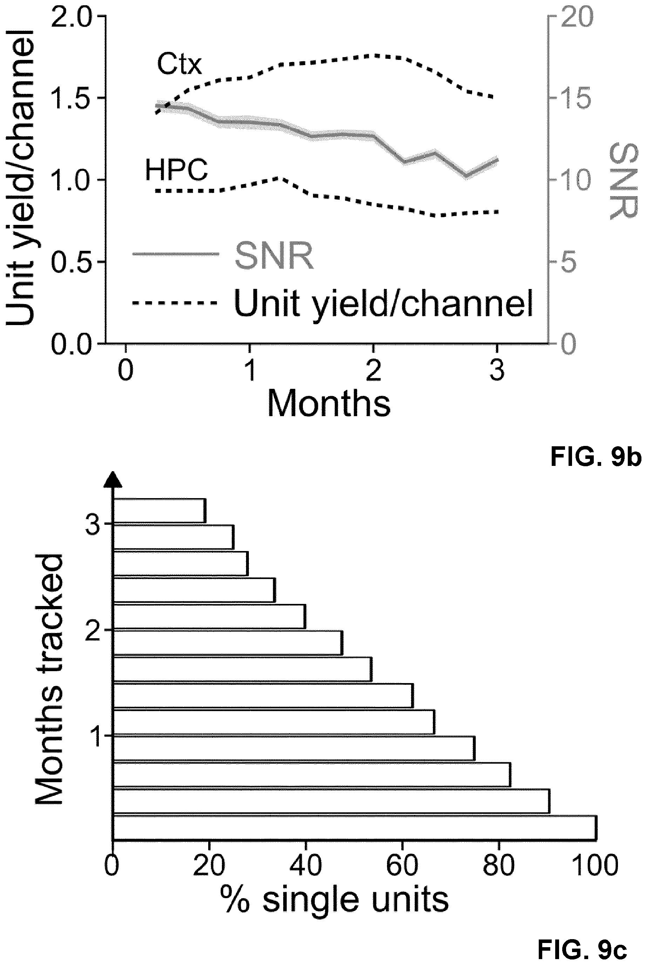

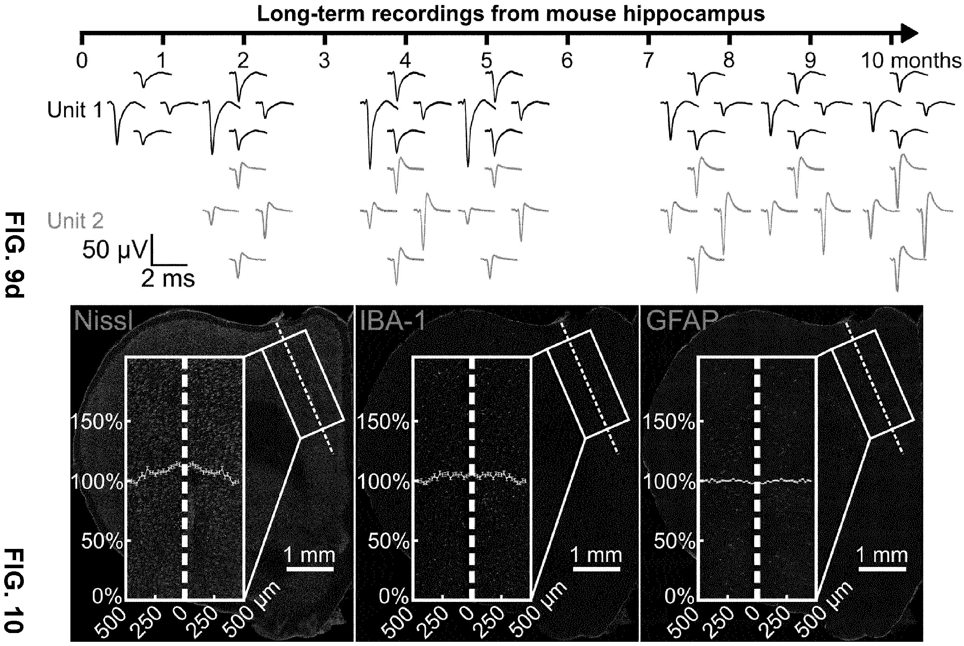

- Fig. 9: A shows: Stability of the single units recorded by the implant device from a rat brain, where the tracking is based on the spike waveforms from multiple releasing-receiving units in the form of electrode sites and the spiking statistics. B: Stability of the unit yield per channel and the signal-to-noise ratio. C: The distribution of single units recorded from rat brains based on the tracking duration by the implant device. D: Two example single units recorded from mouse hippocampus that are tracked for almost a year;
- Fig. 10: shows the immunohistological characterization of the brain tissue implanted for 3.5 months with the implant device;

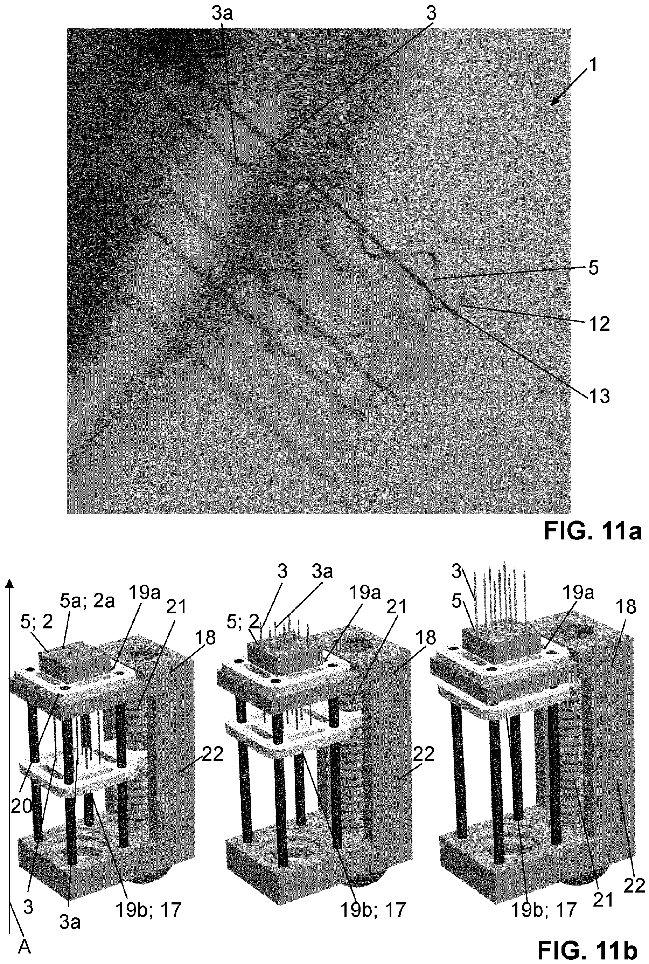

- Fig. 11: A shows: an image of the bundles of fibers in the form of the 2D spiral-shaped precursors being expanded into 3D bundles. B: An alignment device comprising two alignment elements in an initial position (left image), wherein an array of insertion devices is arranged on the lower alignment element and bundles of fibers in the form of the 2D spiral-shaped precursors are arranged on the upper alignment element. Moving the lower alignment element towards the upper alignment element, the insertion devices transform the bundles from the precursor states into the final states (middle and right image);

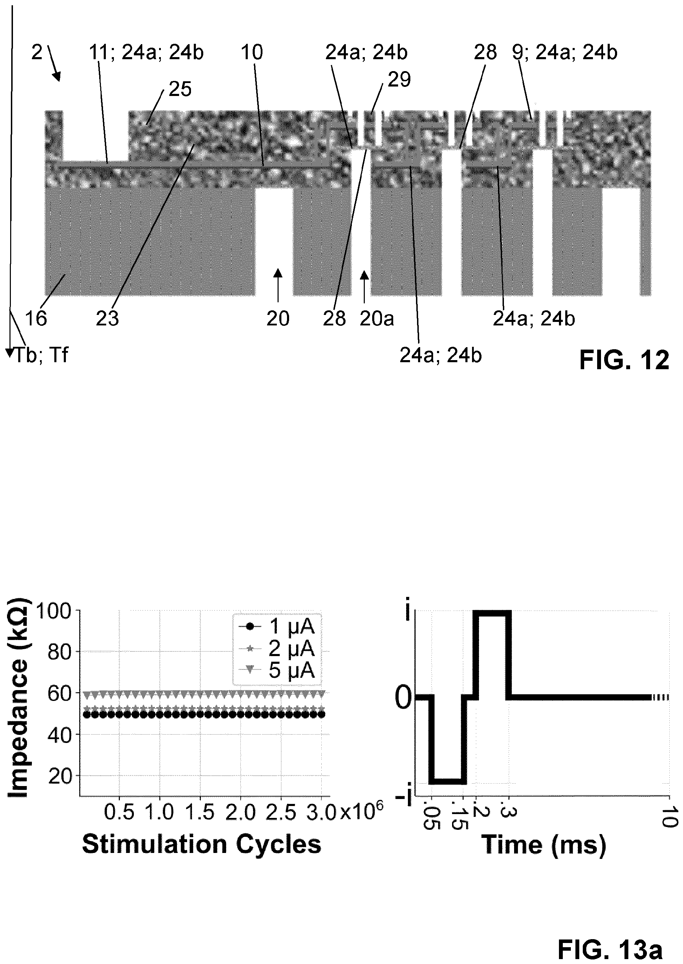

- Fig. 12: shows a sectional view through an implant device comprising spiral-shaped fibers in the precursor state;

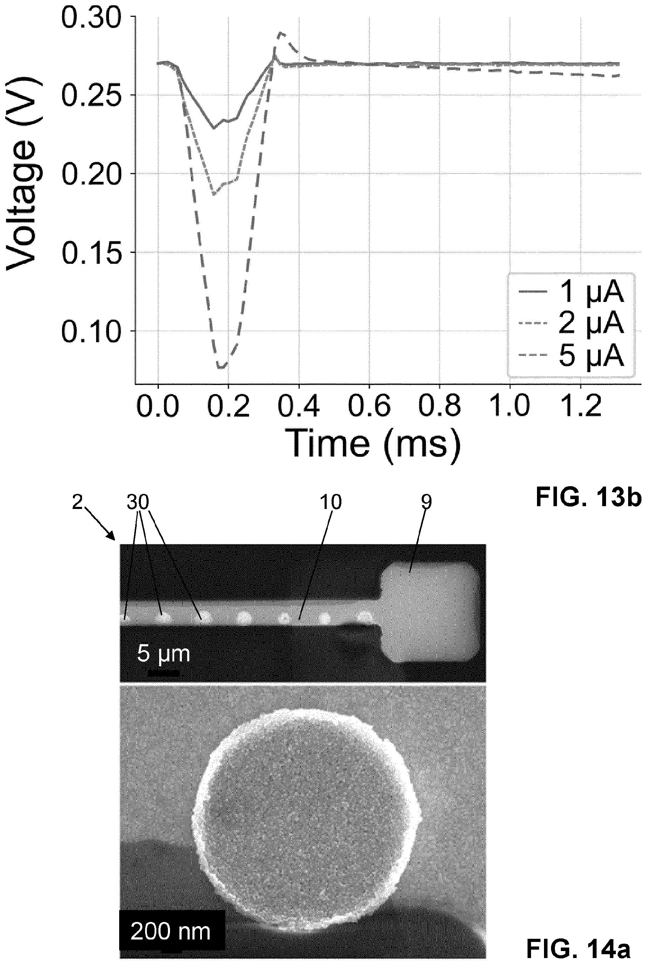

- Fig. 13: A shows: Stability of the electrode impedance magnitudes of releasing-receiving elements in the form of electrodes at 1 kHz (left) after 3 million stimulation cycles (such as shown on the right). B: Voltage-transients measured across the electrode and electrolyte during current injection pulses show that the voltage values staying within safe limits of |V| < 1 V;

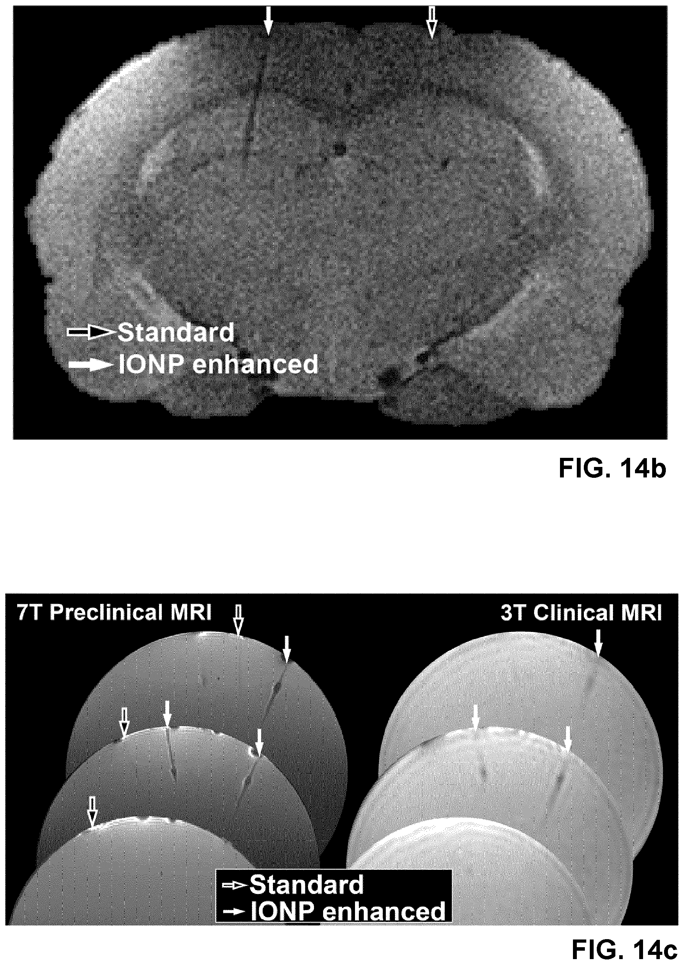

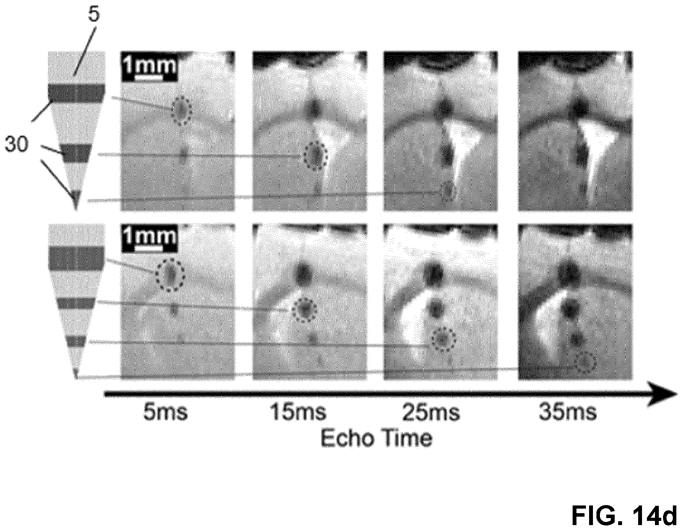

- Fig. 14: A shows: High-resolution patterning of a contrast element in the form of iron-oxide nanoparticles on the transmission elements of the implant device. B: Enhanced contrast of the implant device comprising the contrast element in the form of the iron-oxide nanoparticles in the in vivo magnetic resonance imaging of rat brains. C: Enhanced contrast of the implant device comprising the contrast element in the form of iron-oxide nanoparticles in the 7T pre-clinical and 3T clinical magnetic resonance imaging of agarose sample. D: Two example scenarios with contrast elements in the form of unique identifier patterns of contrast on the bundle of fibers, where the amount and the pattern of the contrast elements can be tuned with respect to imaging needs and parameters.

### DESCRIPTION OF PREFERRED EMBODIMENTS

The present invention discloses a novel geometrical design and manufacturing method for an arrangement 1 comprising three-dimensional (3D) ultra-flexible high-density implant devices 2 for their scalable implantation into biological tissue 4, in particular soft biological tissue. Two main aspects of the arrangement 1 for electrical/optical/chemical interfacing biological tissue are: 1) a scalable three-dimensional assembly of the ultra-flexible implant devices 2 from two-dimensional (2D) precursor state and 2) the minimally invasive, scalable and depth-limitation-free delivery of the ultra-flexible implant devices 2 into the soft, biological tissue.

Various aspects of the arrangement 1 for inserting the implant device 2 into biological tissue 4 are now illustrated with reference to the figures. In fact, the arrangement 1 comprises one or more implant devices 2 and one or more insertion devices 3, wherein the insertion device 3 is configured to penetrate biological tissue 4.The implant device 2 comprises one or more bundles 5 of fibers 6, 6a, ..., wherein in each bundle at least some of the fibers 6, 6a, ... are coupled to one another via a fiber-fiber coupling. Said fiber-fiber coupling can be a temporary or permanent coupling. For instance, the fibers 6, 6a, ... can be temporarily coupled to one another in the fiber-fiber coupling by a coating 7 that coats the fibers, and wherein said coating 7 is biodegradable or configured to dissolve or being digested upon insertion of the implant device 2 into the biological tissue 4 (Fig. 3A to B). Alternatively, the fibers 6, 6a, ... in the fiber-fiber coupling can be permanently coupled to one another via bridging elements 8, 8a,... that tether the fibers 6, 6a, ... to one another, see for instance figure 5a.

The bundle 5 of fibers 6, 6a, ... is transferable from a precursor state into a final state that differs from the precursor state. In the precursor state, the bundle 5 of fibers 6, 6a, ... has a two-dimensional shape, and in the final state, the bundle 5 of fibers 6, 6a, ... has a three-dimensional shape. For example, in the 2D-precursor state of the bundles shown in figures 2a to 2e, the fibers 6, 6a, ... extend parallel to one another and along a longitudinal direction Lb of the bundle 5 of fibers 6, 6a, ... In the 2D-precursor state of the bundles shown in figures 5a and 5b, the fibers 6, 6a, ... are arranged in a curve and have the shape of spirals. In any case, the fibers have a longitudinal shape and have a length along a longitudinal direction Lf of the fiber that is depicted in figure 2A, i.e. the length of the fibers being here 7.2 millimeter. The fibers furthermore have a thickness along a thickness direction Tf of the fiber running perpendicularly to the longitudinal direction Lf of the fiber that is indicated in figure 7. In addition, the fibers have a width along a width direction Wf of the fiber running perpendicularly to the longitudinal direction Lf of the fiber and perpendicularly to the thickness direction Tf of the fiber that is depicted in figure 2c.

As furthermore follows from these figures, the bundle of fibers having a two-dimensional shape means that the fibers are arranged within a (fictitious) two-dimensional plane. The two-dimensional plane preferably is spanned by a first spatial direction and a second spatial direction, the first and second spatial direction running perpendicularly to one another. The first spatial direction can also be referred to as x-direction and the second spatial direction can also be referred to as y-direction.

In the 2D-precursor state of the bundles shown in figures 2a to 2e, the longitudinal direction of the fibers and the width direction of the fibers are arranged or in a sense span said two-dimensional plane. Moreover, the longitudinal direction of the fibers can be assigned to the x-direction and the width direction of the fibers can be assigned to the y-direction, see figure 2c.

In the 2D-precursor state of the bundles shown in figures 5a and 5b, the bundles of fibers 6, 6a, are arranged in a two-dimensional array and in a two-dimensional plane that is defined here by a fiber-support element 16 or a silicon die the fibers are manufactured on. In particular, the fiber-support element 16 or die extends along x-y directions that span the two-dimensional plane, see figure 5a.

The fibers, temporarily or permanently tethered to each other in the fiber-fiber coupling, are then expanded into a 3D bundle or multiple bundles. Since these bundles are too flexible to be inserted into the tissue independently, mechanical support from a stiff insertion device 3 is required during the insertion process.

In particular, the implant device 2 and the insertion device 3 are configured to couple to one another via an implant-insertion coupling. When being coupled to the insertion device 3 in the implant-insertion coupling, the implant device 2 is insertable into the biological tissue 4 via the insertion device 3. To provide this, the longest fiber (designated the deepest point in its respective bundle) can have a coupling element 12 in the form of a loop at its tip (Fig. 2B). This loop 12 is used for tethering to a coupling element 13 of the insertion device 3 with sufficient stiffness to penetrate into the targeted depth in the tissue in an implant-fiber coupling (Fig. 3C). The coupling element 13 of the insertion device 3 can be provided by the tip of the insertion device 3 being shaped to match the coupling element 12 in the form of the loop at the end of the implant device 2, in particular at the terminal end 14 of the fibers, enabling a robust mechanical tethering between the two (Fig. 4).

As best seen in figures 3c and 4, the insertion device 3 is an elongated device extending along a longitudinal direction Li. In the depicted examples, a length of the insertion device 3 along the longitudinal direction Li of the insertion device is 8 mm. A width or diameter of the insertion device along a width direction Wi of the insertion device 3 running perpendicularly to the longitudinal direction Li of the insertion device and perpendicularly to the thickness direction Ti of the insertion device is 50 micrometers. The coupling element 13 of the insertion device 3 in the form of the tip or needle is formed here by a steep taper of the insertion device 3 when seen inwards in the direction of a central longitudinal axis Ci of the insertion device 3 running parallel to the longitudinal axis Li of the insertion device 3, which ends in the terminal end 15 of the insertion device (Fig. 3C).

Hence, several designs of the implant devices and their 2D precursor states are provided. The choice of the precursor shape and how the fibers are tethered to each other determines the strategies for the expansion of the 2D precursors into 3D bundles and the insertion of the 3D bundles into the soft biological tissue. For instance, the fibers 6, 6a, ... are entirely independent once released from an underlying substrate such as a fiber-support element 16 used as a carrier during the fabrication of the implant device 2 (Fig. 2E), see also explanations further below. Upon immersion into an aqueous medium and subsequent extraction, the fibers 6, 6a, ... coalesce into bundles through elastocapillary forces (Fig. 3A).

This bundle 5 comprises a temporary fiber-fiber coupling that is provided by a biodegradable chemical coating 7 (e.g., silk fibroin, polyethylene glycol, polylactic acid) of the fibers (Fig. 3B). Once the bundle 5 of fibers 6, 6a, ... is inserted into the soft biological tissue, the coating 7 is dissolved/digested (Fig. 4). This leaves the fibers 6, 6a, ... mechanically independent of each other, granting them maximal mechanical compliance with the soft brain tissue (Fig. 1A).

Alternatively, the fibers 6, 6a, .... are shaped as curved shapes, such as spirals, with the coupling element 12 in the form of the loop of the deepest fiber 6 in the center of these shapes. The bundles 5, 5a, ... designated to be adjacent to each other inside the tissue form a grid of 2D precursors before they are released from the fiber-support element 16 they are carried on during the manufacturing process (Fig. 5A, B). In this case, all precursors are simultaneously expanded into 3D bundles by an array of stiff insertion devices 3, 3a, ... being arranged or formed on an insertion-support element 17 by aligning the coupling elements 13, 13a, ... in the form of the tips of the stiff insertion devices 3, 3a, ... with the coupling elements 12, 12a, ... in the form of the loops of the implant devices 2, 2a, ... at the centers of the precursors (Fig. 5B). This arrangement further allows the bundles 5, 5a, ... to be inserted parallelly into the soft tissue (Fig. 5C).

As follows from these figures, the plurality of bundles 5, 5a, in their precursor states are arranged in a common plane and in a two-dimensional array. Said common plane is here defined by a fiber-support element 16. As will be explained with reference to figure 12, said fiber-support element 16 preferably corresponds to a die of a wafer on which the fibers are fabricated, in particular patterned on.

Figure 6 depicts fibers 6, 6a, ... being coupled to one another in the fiber-fiber coupling via the bridging elements 8, 8a, .... In this design, there is a small (<1:100 length ratio compared to the entire length of the fibers) mechanical bridge that tethers the fibers 6, 6a, ... to one another. In this case, no coating is required to keep the bundle 5 together since the mechanical bridges provided by the bridging elements 8, 8a, ... between the fibers 6, 6a, ... serve that purpose.

Due to the extreme flexibility and the small size of the fibers, they provide maximal compliance with the brain tissue, stable recordings of brain activity, and no observable chronic damage to the brain tissue as seen in stiff electrode arrays. Compared to the upcoming flexible electrode technologies mentioned above, our invention allows the scalable implanting of ultra-flexible electrode fibers anywhere in the brain at high density in three dimensions and with minimal disruption to the brain tissue. The present method provides a higher density of releasing-receiving elements to be packed in a smaller footprint than the flexible electrodes of the prior art (Fig. 1A), where flexible shanks are inserted by mechanical tethering to stiff insertion devices. Furthermore, the present invention does not have a limitation on the implantation depth, as seen in the flexible electrodes where flexible shanks or bundles of thin fibers are inserted into the brain by chemical tethering to stiff insertion device (Fig. 1B).

Moreover, each fiber 6, 6a, .... comprises one or multiple releasing-receiving elements 9, an input-output element 11 to an external device, and a transmission element 10 that serves the connection between the releasing-receiving elements 9 and the input-output element, see for instance figures 2a to 2c. Depending on the purpose of the implant devices 2, the releasing-receiving elements 9 can be used for the measurement of electrical activity or fluorescence in the biological tissue, stimulation of the tissue with electrical currents or light, measurement of the concentrations of chemicals in the tissue, or the delivery of chemicals into the tissue.

The implant device 2, in particular the fibers 6, 6a, ..., comprises a flexible material such as a polymer and at least one metallic compound such as a metallic layer that provide the releasing-receiving elements 9, 9a, input-output elements 11, and transmission elements 10. In particular, the releasing-receiving elements 9, 9a, input-output elements 11, and transmission elements 10 were defined by titanium film 24b /gold layer 24a patterned on a substrate layer of polyimide (Fig. 7A). The titanium/gold layer was encapsulated in a thin, insulating layer 25 of polyimide (Fig. 7B). The insulating polyimide layer was etched above the input-output elements 11 and at the outlines 26, 26a of the fibers, bridging elements (if applicable) and coupling elements (Fig. 7C). The electrical connection from the releasing-receiving elements 9, 9a to the input-output elements 11 can partially consist of multiple layers of metal that are isolated from each other with thin layers of polymer film, by the repetition of the steps demonstrated in Fig. 7A, 7B and 7C. The releasing-receiving elements 9, 9a are coated with a impedance-reducing coating layer 27 (e.g., PEDOT:PSS) that reduces the electrical impedance between the electrode site and the surrounding tissue (Fig. 7G). This impedance reduction enables higher signal-to-noise ratios during recordings of neural activity and higher charge-injection capacities during brain stimulation. The impedance-reducing coating layer 27 was done by coating the implant devices with a sacrificial layer 28 of parylene C (Fig. 7D), plasma etching of the parylene C 28 and polyimide above the releasing-receiving elements 9, 9a (Fig. 7E), spin-coating of the implant devices with PEDOT:PSS (Fig. 7F), and removal of the sacrificial parylene layer 28 (Fig. 7G).

In the following, a concrete example of manufacturing an implant device according to the invention is given. In particular, in a first step PI2610 (HD Microsystems) is spun on a 4-inch silicon wafer to form a 1.2 µm thick polyimide layer. After curing the polyimide layer 23 in a programmable oven (CLO-2AH-S, Koyo ThermoSystems) at 300°C, we patterned man-1420 (Micro Resist Technology GmbH) on this polyimide layer 23 by direct laser lithography (Heidelberg Instruments DWL 66+). The releasing-receiving elements 9, 9a, ..., input-output elements 11, 11a, ..., and transmission elements 10, 10a, ... in the form of solder pads, electrode contacts, and wires were patterned by lift-off after depositing 10 nm titanium and 150 nm gold with electron-beam evaporation (Plassys MEB550S). We coated the metal layer with a 1.2 µm-thick polyimide insulating layer 25 and patterned AZP4620 (MicroChemicals GmbH) on it using direct laser lithography to define the canals between electrode fibers 6, 6a, ..., the device borders, the openings above the input-output elements 11, 11a, ..., the bridging elements (if applicable) and the loops 12, 12a, ... at the tips of the bundles 5, 5a, ... that serve as the coupling elements. We etched the polyimide in exposed areas in O₂/CF₄ plasma (Plasmalab 80 Plus, Oxford Instruments) and removed the excess photoresist.

To pattern PEDOT:PSS impedance-reducing coating layer 27 on the electrode contacts via a dry lift-off process, we coated a 2.5 µm layer of sacrificial parylene C layer 28 on the wafer by chemical vapor deposition (PDS 2010 Labcoter 2; Specialty Coating Systems Inc.). We spun a layer of 2% Micro-90 solution (International Products Corporation) on the wafer before parylene coating to facilitate the separation of the sacrificial parylene later. Afterwards, we patterned AZP4620 on the parylene C by direct laser lithography, coating everything except the recording contacts 9, 9a, .... Then, we etched the parylene C and polyimide on the recording contacts 9, 9a, ... in O₂/CF₄ plasma. We placed the wafer briefly inside a diluted gold etchant (KI/I₂, FIRST Micro- and Nanotechnology Center, ETH Zurich) to roughen the gold on the recording contacts 9, 9a, ... and enhance the adhesion of the PEDOT:PSS impedance-reducing coating layer 27 to the electrode contacts 9, 9a, .... We rinsed the wafer in ultrapure water and removed the excess photoresist. We spun a mixture of PEDOT:PSS (Clevios PH 1000, Heraeus Epurio), ethylene glycol (Sigma Aldrich), dodecyl benzene sulfonic acid (Sigma Aldrich), and (3-Glycidyloxypropyl) trimethoxysilane (Sigma Aldrich) on the wafer at 650 RPM, and placed the wafer in an oven at 140°C for one hour. We repeated the spinning/baking of the PEDOT:PSS mixture two more times to achieve a total thickness of 450 nm. After the wafer cooled down, we peeled off the parylene C sacrificial layer 28, leaving PEDOT:PSS only on recording contacts.

The successful assembly and implantation of the bundles 5, 5a, ... comprising mechanically independent fibers 6, 6a, ... in multiple cortical and sub-cortical areas of rat and mouse brains were demonstrated. In this implementation, metallic wires, in particular tungsten wires, with a 50 µm diameter were used as the stiff insertion devices 3, 3a, ... and silk fibroin as the coating 7 of the fiber-fiber coupling for the electrode bundle 5, 5a, .... These implant devices 2, 2a, ... allowed the recording of high-quality single-neuron and population activity from multiple brain areas (Fig. 8). Furthermore, single neurons can be tracked for almost a year due to the extreme flexibility of the electrode fibers (Fig. 9). Immunohistochemical assessment of the implanted brain tissues showed no significant chronic damage to the brain tissue surrounding the electrode bundles 5, 5a, ... (Fig. 10).

The successful parallel expansion of an array of spiral-shaped electrode fibers 6, 6a, ... into an array of 3D electrode bundles were also demonstrated (Fig. 11A). In particular, and as follows from figure 11b, the arrangement 1 comprises an alignment device 18 that in turn comprises two alignment elements 19a, 19b being arranged above one another with respect to an alignment direction A. The insertion device 3 is connectable to the lower alignment elements 19a and the implant device 2 comprising the bundle 5 of fibers 6, 6a, ... in the precursor state is connectable to the upper alignment element 19b.

The alignment elements 19a, 19b are mounted to a frame of the alignment device and are movable with respect to one another and along the alignment direction A and along a coupling direction along which the implant devices 2, 2a, ... and the insertion devices 3, 3a, ... couple to another such, that the insertion devices 3, 3a, ... couple to the implant devices 2, 2a, ... in the implant-insertion coupling and transfer the bundles 5, 5a, ... of fibers 6, 6a, ... from the precursor state into the final state. The alignment device 18 further comprises a driving element 21 here in the form of a screw that is configured to drive the movement of the alignment elements 19a, 19b.

Moreover, the alignment element 19a connectable to the implant device 2 comprises guiding holes 20, 20a, ... that are arranged to match the position of the insertion devices 3, 3a, ... when being connected to their alignment element 19b such, that the insertion devices 3 partially protrude though the respective guiding holes 20 upon moving here the lower alignment element 19a towards the upper alignment element 19b, whereby the insertion devices 3, 3a, ... couple to the bundles 5, 5a, ... of fibers 6, 6a, ... in the implant-insertion coupling and transfer the bundles 5, 5a, ... from the precursor states into the final states in a guided manner.

In the depicted example, an array of insertion devices 3, 3a, ... in the form of tungsten wires were used, where the wires are moved on a single axis along the alignment direction A by using the driving element 21 (Fig. 11B) to expand the 2D precursors to 3D electrode bundles 5, 5a, .... In this tungsten wire array, the wires 3, 3a, ... are fixed to the bottom alignment element 19b and can be freely moved through the top alignment element 19a. Here, said bottom alignment element 19b corresponds to an insertion-support element 17 that serves the purpose of supporting the insertion devices 3, 3a, ... During the expansion process, the insertion devices 3, 3a, ... in the form of the tungsten wires are fully retracted below the surface of the top alignment element 19a. The fiber-support element 16 is aligned with and positioned on the surface of the top alignment element. Afterwards, the bottom alignment element is moved upwards to push the insertion devices in the form of the tungsten wires out of the surface of the top alignment element, which results in the engagement of the coupling elements in the form of the loops of the spirally shaped bundles of fibers with the array of the tungsten wires and the expansion of the 2D spiral precursors into 3D electrode bundles (Fig. 11A-11C). The tungsten wire array can then be inserted into the brain to deliver the electrode bundles in the brain, as in the other electrode designs mentioned above.

The 2D precursors of the implant device 2 can also comprise additional layers to facilitate the alignment of the bundles 5, 5a, ... of fibers 6, 6a, ... with the array of stiff insertion devices 3, 3a, ... and the 2D to 3D expansion process (Fig. 12), such as a fixation layer, e.g. an aluminium layer, being arranged between the fiber-support element 16 that is here provided in the form of a silicon layer or silicon die and the further components of the implant device 2 such as the substrate layer 23 and insulating layer 25 of polyimide to keep them attached to the underlying fiber-support element 16 before the expansion of the bundles 5, 5a, ... of fibers 6, 6a, .... There are also photolithographically defined guiding holes 20, 20a, ... in the underlying fiber-support element 16 or substrate layer 23, which are etched with deep reactive ion etching that enables precise positioning of the coupling elements 13, 13a, ... in the form of the tips of the stiff insertion devices 3, 3a, ... with respect to the centers 29 of the bundles 5, in particular or the spirals, in the 2D precursor states.

In addition to the recording capabilities, the stimulation capabilities of the implant device 2 have been tested. The magnitudes of the impedances of the releasing-receiving elements 9 at 1 kHz were relatively stable after millions of current injection pulses (Fig. 13A). Furthermore, the voltage transients measured across the releasing-receiving elements 9 and the electrolyte remained within the safe limits of greater than -1 V and less than 1 V during the current injection pulses (Fig. 13B).

In order to enhance the visibility of the implant devices 2, 2a, ... implanted in the biological tissue, a contrast element 30 such as iron oxide nanoparticles can be patterned on the transmission elements 10 or releasing-receiving elements 9 with high resolution (Fig. 14A). As a result, the implant devices 2, 2a, ... with iron oxide nanoparticles become visible during in vivo 7T pre-clinical magnetic resonance imaging, whereas the implant devices without nanoparticles are invisible (Fig. 14B). Furthermore, this contrast enhancement was also achieved in 3T magnetic resonance imaging, which is typically used for clinical purposes (Fig. 14C).

The contrast element can be deposited in the form of a unique identifier pattern on the precursor of the implant device 2. In the example depicted in figure 14a, the contrast element is provided in the form of a unique identifier pattern consisting of dots. The unique identifier pattern of contrast distinguishes each implant device when multiple implant devices are in the biological tissue. The implant devices can be placed in the biological tissue with 300 micrometers of lateral separation and still can be identified. The amount and the pattern of the contrast element can be tuned with respect to imaging conditions and parameters to achieve the desired distinguishability and precision (Fig. 14D).

### LIST OF REFERENCE SIGNS

- 1: arrangement
- 2: implant device
- 3: insertion device
- 4: biological tissue
- 5: bundle
- 6, 6a,: fibers
- 7: coating
- 8, 8a,: bridging element
- 9: releasing-receiving element
- 10: transmission element
- 11: input-output element
- 12: coupling element of implant device
- 13: coupling element of insertion device
- 14, 14a,: terminal end of fibers
- 15, 15a,: terminal end of insertion device
- 16: fiber-support element
- 17: insertion-support element
- 18: alignment device
- 19a, 19b: alignment element
- 20: guiding hole
- 21: driving element
- 22: frame
- 23: substrate layer
- 24a, 24b: metallic layer
- 25: insulating layer
- 26: outline
- 27: impedance-reducing coating layer
- 28: sacrificial layer
- 29: center of the bundle
- 30: contrast element

- Lb: longitudinal direction of bundle
- Lf: longitudinal direction of fiber
- Li: longitudinal direction of insertion device
- Tf: thickness direction of fiber
- Wf: width direction of fiber
- Wi: width direction of insertion device
- Ti: thickness direction of insertion device
- Ci: central longitudinal axis of insertion device
- A: alignment direction

## Claims

1. An arrangement (1) for inserting an implant device (2) into biological tissue (4) comprising:
- at least one implant device (2), and
- at least one insertion device (3),
wherein the insertion device (3) is configured to penetrate biological tissue (4),
wherein the implant device (2) and the insertion device (3) are configured to couple to one another via an implant-insertion coupling,
wherein the implant device (2), when being coupled to the insertion device (3) via the implant-insertion coupling, is insertable into the biological tissue (4) via the insertion device (3),
**characterized in that** the implant device (2) comprises at least one bundle (5) of fibers (6, 6a, ...),
wherein the fibers (6, 6a, ...) are coupled to one another via a fiber-fiber coupling, and
wherein the implant-insertion coupling is different from the fiber-fiber coupling.

2. The arrangement (1) according to claim 1, wherein in the fiber-fiber coupling:
- the fibers (6, 6a, ...) are temporarily coupled to one another and/or the bundle of fibers (6, 6a, ...) are at least partially coated with at least one coating (7), the coating (7) being biodegradable and/or being configured to dissolve and/or being digested upon insertion of the implant device (2) into the biological tissue (4), or
- the fibers (6, 6a, ...) are permanently coupled to one another and/or the fibers (6, 6a, ...) comprise one or more bridging elements (8, 8a, ...) tethering the fibers (6, 6a, ...) to one another, the bridging elements (8, 8a, ...) preferably comprising or consisting of at least one polymer.

3. The arrangement (1) according to any one of the preceding claims, wherein the bundle (5) of fibers (6, 6a, ...) is transferable from a precursor state into a final state that differs from the precursor state, and
wherein in the precursor state, the bundle (5) of fibers (6, 6a, ...) has a two-dimensional shape, and in the final state, the bundle (5) of fibers (6, 6a, ...) has a three-dimensional shape, and/or
wherein the implant device (2) is insertable into the biological tissue (4) via the insertion device (3) in the implant-insertion coupling with the bundle (5) of fibers (6, 6a, ...) being in the final state.

4. The arrangement (1) according to claim 3, wherein, in the precursor state, the fibers (6, 6a, ...) extend parallel to one another and/or along a longitudinal direction (Lb) of the bundle (5) of fibers (6, 6a, ...), or
wherein, in the precursor state, the fibers (6, 6a, ...) are arranged in a whorled pattern such as a spiral.

5. The arrangement (1) according to claim 3 or 4, wherein the bundle (5) of fibers (6, 6a, ...) is transferable from the precursor state into the final state via at least one of:
- the insertion device (3), preferably upon coupling of the insertion device (3) to the bundle (5) of fibers (6, 6a, ...) in the implant-insertion coupling and movement of the insertion device (3) with respect to the implant device (2),
- a movement of the fibers (6, 6a, ...), in particular a deformation of the fibers (6, 6a, ...), or
- one or more forces generated by the fibers (6, 6a, ...) and/or one or more forces acting on the fibers (6, 6a, ...), in particular via elastocapillary forces generatable by the fibers (6, 6a, ...) and acting on the fibers (6, 6a, ...).

6. The arrangement (1) according to any one of the preceding claims, wherein the bundle (5) of fibers (6, 6a, ...) comprises at least one of:
- at least one releasing-receiving element (9), wherein the releasing-receiving element (9) is configured to release into the biological tissue (4) at least one of: a signal being configured to stimulate the biological tissue (4), a diagnostic agent, or a therapeutic agent and/or wherein the releasing-receiving element (9) is configured to receive a signal being associated with the biological tissue (4), or
- at least one transmission element (10) being configured to transmit at least one of signals, diagnostic or therapeutic agents between i) a releasing-receiving element (9) and ii) at least one input-output element (11), the input-output element (11) being configured to input at least one of: signals, diagnostic or therapeutic agents into the transmission element (10) and to output signals from the transmission element (10) to an external device being configured to generate or collect signals, diagnostic or therapeutic agents.

7. The arrangement (1) according to claim 6, wherein:
- the signal being released into the biological tissue (4) and the signal being associated with the biological tissue (4) are electrical signals, the releasing-receiving element (9) and the input-output element (11) being a resistive or capacitive element, and the transmission element (10) being an electrically conducting element; or
- the signal being released into the biological tissue (4) and the signal being associated with the biological tissue (4) are optical signals, the transmission element (10) being i) a waveguide configured to transmit optical signals or ii) an electrically conducting element that is configured to transmit electrical signals to drive a light-emitting element of the releasing-receiving element (9), or
- the releasing-receiving element (9) being an outlet for releasing diagnostic or therapeutic agents into the biological tissue (4) and/or the releasing-receiving element (9) being a sensor element for sensing an amount of diagnostic or therapeutic agents in the biological tissue (4) and/or the transmission element (10) being a microfluidic channel or an electrically conducting element.

8. The arrangement (1) according to any one of the preceding claims, wherein the implant device (2), in particular the fibers (6, 6a, ...), comprises or consists of at least one of: at least one flexible material, at least one polymer, at least one metallic compound such as a metallic layer or combinations thereof, and/or
wherein the implant device (2), in particular the fibers (6, 6a, ...), comprises at least one contrast element (30) being configured to generate or enhance a contrast in the biological tissue (4) during imaging of the biological tissue (4) with electromagnetic waves, wherein the contrast element (30) is preferably provided as an unique identifier pattern specific to the implant device such, that the implant device in the biological tissue during imaging with electromagnetic waves can be identified.

9. The arrangement (1) according to any one of the preceding claims, wherein the insertion device (3) is at least one of:
- stiff,
- retractable from the implant device (2) after insertion of the implant device (2) into the biological tissue (4) or dissolvable upon the insertion of the implant device (2) into the biological tissue (4),
- water-soluble or water-insoluble,
- comprising or consisting of at least one of: at least one metal compound, at least one non-metal compound, at least one metalloid compound, at least one ceramics compound, at least one biodegradable compound, at least one polymer or mixtures thereof.

10. The arrangement (1) according to any one of the preceding claims, wherein the implant device (2) comprises at least one coupling element (12) and the insertion device (3) comprises at least one coupling element (13) that are configured to couple to one another in the implant-insertion coupling, and wherein:
- the coupling element (12) of the implant device (2) and the coupling element (13) of the insertion device (3) are configured to mechanically couple to one another, and/or
- the coupling element (12) of the implant device (2) is arranged and/or integrally formed on the bundle (5) of fibers (6, 6a, ...), in particular at a terminal end (14, 14a, ...) of a fiber (6, 6a, ...), and/or
- the coupling element (13) of the insertion device (3) is arranged and/or integrally formed on the insertion device (3), in particular at a terminal end (15, 15a, ...) of the insertion device (3).

11. The arrangement (1) according to any one of the preceding claims, wherein the implant device (2) comprises a plurality of bundles (5, 5a, ...) of fibers (6, 6a, ...), and
wherein at least in precursor states of the plurality of bundles of fibers (6, 6a, ...), the plurality of bundles (5, 5a, ...) of fibers (6, 6a, ...) are arranged at least one of:
- on a fiber-support element (16),
- in a common plane, or
- in a one-dimensional array or in two-dimensional array.

12. The arrangement (1) according to any one of the preceding claims, the arrangement (1) comprising a plurality of insertion devices (3, 3a, ...), and
wherein a plurality of bundles (5, 5a, ...) of fibers (6, 6a, ...) is transferrable from precursor states into final states via the plurality of insertion devices (3, 3a, ...) and preferably simultaneously, and/or
wherein the plurality of insertion devices (3, 3a, ...) is arranged at least one of:
- on an insertion-support element (17),
- in a common plane, or
- in a one-dimensional array or in a two-dimensional array.

13. The arrangement (1) according to any one of the preceding claims, comprising at least one alignment device (18) that comprises at least two alignment elements (19a, 19)b being arranged above one another with respect to an alignment direction (A),
wherein the insertion device (3) is connectable to one of the alignment elements (19a) and the implant device (2) comprising the bundle (5) of fibers (6, 6a, ...) in the precursor state is connectable to the other alignment element (19b), and
wherein the alignment elements (19a, 19b) are movable with respect to one another and along the alignment direction (A) such, that the insertion device (3) transfers the bundle (5) of fibers (6, 6a, ...) from the precursor state into the final state.

14. The arrangement (1) according to claim 13, wherein at least one of:
- the alignment element (19a) being connectable to the implant device (2) comprises at least one guiding hole (20) that is arranged to match a position of the insertion device (3) when being connected to its alignment element (19b) such, that the insertion device (3) is at least partially protruding though the guiding hole (20) upon moving the alignment elements (19a, 19b) with respect to one another, whereby the insertion device (3) is configured to transfer the bundle (5) of fibers (6, 6a, ...) from the precursor state into the final state in a guided manner, or
- the alignment device (18) further comprises at least one driving element (21) being configured to drive the movement of the alignment elements (19a, 19b), the driving element (21) preferably being a mechanical driving element such as a screw or an electrical driving element such as a piezoelectric element or a motor.

15. A method of manufacturing an arrangement (1) for inserting an implant device (2) into biological tissue (4), preferably the arrangement (1) according to any one of the preceding claims, the method comprising the steps of:
- Providing at least one implant device (2), and
- Providing at least one insertion device (3),
wherein the insertion device (3) is configured to penetrate biological tissue (4),
wherein the implant device (2) and the insertion device (3) are configured to couple to one another via an implant-insertion coupling,
wherein the implant device (2), when being coupled to the insertion device (3) via the implant-insertion coupling, is insertable into the biological tissue (4) via the insertion device (3),
**characterized in that** the implant device (2) comprises at least one bundle (5) of fibers (6, 6a, ...), and
wherein the fibers (6, 6a, ...) are coupled to one another via a fiber-fiber coupling, and
wherein the implant-insertion coupling is different from the fiber-fiber coupling.
